# EUROPÄISCHE PATENTSCHRIFT

(11) **EP 2 600 889 B1**
(45) Veröffentlichungstag und Bekanntmachung des Hinweises auf die Patenterteilung: **29.11.2017**
(21) Anmeldenummer: 11743999.2
(22) Anmeldetag: 05.08.2011
(51) Int. Cl.: A61K 39/00, C07K 16/28, C07K 16/30

(54) **ANTI-LA ANTIKÖRPER UND IHRE ANWENDUNG ZUM IMMUNOTARGETING**
ANTI-LA ANTIBODIES AND THEIR USE FOR IMMUNOTARGETING
ANTICORPS ANTI-LA ET UTILISATION POUR UN IMMUNOCIBLAGE

(30) Priorität: 06.08.2010 DE 102010039018
(43) Veröffentlichungstag der Anmeldung: 12.06.2013
(73) Patentinhaber: GEMoaB Monoclonals GmbH, 01307 Dresden (DE)
(72) Erfinder: BACHMANN, Michael, 65779 Kelkheim (DE)
(74) Vertreter: Kailuweit & Uhlemann Patentanwälte Partnerschaft mbB
(86) Internationale Anmeldenummer: PCT/EP2011/063539
(87) Internationale Veröffentlichungsnummer: WO 2012/017082

(56) Entgegenhaltungen:
- US-A1- 2005 136 050
- BAEUERLE PATRICK A ET AL: "Bispecific T-cell engaging antibodies for cancer therapy.", CANCER RESEARCH 15 JUN 2009 LNKD- PUBMED:19509221, Bd. 69, Nr. 12, 15. Juni 2009 (2009-06-15) , Seiten 4941-4944, XP002665118, ISSN: 1538-7445
- BAEUERLE PATRICK A ET AL: "BiTE: Teaching antibodies to engage T-cells for cancer therapy", CURRENT OPINION IN MOLECULAR THERAPEUTICS, THOMSON REUTERS (SCIETIFIC) LTD, Bd. 11, Nr. 1, 1. Februar 2009 (2009-02-01), Seiten 22-30, XP009151509, ISSN: 2040-3445 in der Anmeldung erwähnt
- BARGOU RALF ET AL: "Tumor regression in cancer patients by very low doses of a T cell-engaging antibody", SCIENCE, AMERICAN ASSOCIATION FOR THE ADVANCEMENT OF SCIENCE, WASHINGTON, DC; US, Bd. 321, Nr. 5891, 15. August 2008 (2008-08-15), Seiten 974-977, XP002615656, ISSN: 0036-8075, DOI: 10.1126/SCIENCE.1158545 in der Anmeldung erwähnt
- AL-EJEH F ET AL: "The La Autoantigen Is a Malignancy-Associated Cell Death Target That is Induced by DNA-Damaging Drugs", CLINICAL CANCER RESEARCH, THE AMERICAN ASSOCIATION FOR CANCER RESEARCH, US, Bd. 13, Nr. 18, SUPPL, 15. September 2007 (2007-09-15), Seiten 5509S-5518S, XP008105261, ISSN: 1078-0432, DOI: 10.1158/1078-0432.CCR-07-0922 in der Anmeldung erwähnt
- AL-EJEH F ET AL: "In vivo Targeting of Dead Tumor Cells in a Murine Tumor Model Using a Monoclonal Antibody Specific for the La Autoantigen", CLINICAL CANCER RESEARCH, THE AMERICAN ASSOCIATION FOR CANCER RESEARCH, US, Bd. 13, Nr. 18, SUPPL, 15. September 2007 (2007-09-15), Seiten 5519S-5527S, XP008105259, ISSN: 1078-0432, DOI: 10.1158/1078-0432.CCR-07-0964 in der Anmeldung erwähnt
- BACHMANN M ET AL: "Translocation of the nuclear autoantigen La to the cell surface of herpes simplex virus type 1 infected cells.", AUTOIMMUNITY 1992 LNKD- PUBMED:1617103, Bd. 12, Nr. 1, 1992, Seiten 37-45, XP009154578, ISSN: 0891-6934
- KORISTKA STEFANIE ET AL: "Retargeting of regulatory T cells to surface-inducible autoantigen La/SS-B", JOURNAL OF AUTOIMMUNITY, LONDON, GB, vol. 42, 22 January 2013 (2013-01-22), pages 105-116, XP028531549, ISSN: 0896-8411, DOI: 10.1016/J.JAUT.2013.01.002
- WANG W W ET AL: "Antigen targeting to dendritic cells with bispecific antibodies", JOURNAL OF IMMUNOLOGICAL METHODS, ELSEVIER SCIENCE PUBLISHERS B.V.,AMSTERDAM, NL, vol. 306, no. 1-2, 30 November 2005 (2005-11-30), pages 80-92, XP027659256, ISSN: 0022-1759 [retrieved on 2005-11-30]
- SCHMITZ MARC ET AL: "Tumoricidal potential of native blood dendritic cells: Direct tumor cell killing and activation of NK cell-mediated cytotoxicity", THE JOURNAL OF IMMUNOLOGY, THE AMERICAN ASSOCIATION OF IMMUNOLOGISTS, US, vol. 174, no. 7, 1 April 2005 (2005-04-01) , pages 4127-4134, XP002629625, ISSN: 0022-1767
- PROSKURYAKOV SERGEY Y ET AL: "Mechanisms of tumor cell necrosis.", CURRENT PHARMACEUTICAL DESIGN JAN 2010, vol. 16, no. 1, January 2010 (2010-01), pages 56-68, ISSN: 1873-4286
- B J Kroesen ET AL: "Bispecific antibodies for treatment of cancer in experimental animal models and man", Advanced Drug Delivery Reviews, vol. 31, 1 January 1998 (1998-01-01), pages 105-129, XP055299943, DOI: 10.1016/S0169-409X(97)00096-3
- CHAMES PATRICK ET AL: "Bispecific antibodies for cancer therapy. The light at the end of the tunnel?", MABS, LANDES BIOSCIENCE, US, vol. 1, no. 6, 1 November 2009 (2009-11-01), pages 539-547, XP002688758, ISSN: 1942-0862, DOI: 10.4161/MABS.1.6.10015
- GONZALEZ S ET AL: "NKG2D ligands: key targets of the immune response", TRENDS IN IMMUNOLOGY, ELSEVIER LTD. * TRENDS JOURNALS, GB, vol. 29, no. 8, 1 August 2008 (2008-08-01) , pages 397-403, XP023181111, ISSN: 1471-4906, DOI: 10.1016/J.IT.2008.04.007 [retrieved on 2008-07-03]
- KUROKI MASAHIDE ET AL: "RE-TARGETING OF CYTOTOXIC T LYMPHOCYTES AND/OR NATURAL KILLER CELLS TO CEA-EXPRESSING TUMOR CELLS WITH ANTI-CEA ANTIBODY ACTIVITY", ANTICANCER RESEARCH - INTERNATIONAL JOURNAL OF CANCER RESEARCH AND TREAT, INTERNATIONAL INSTITUTE OF ANTICANCER RESEARCH, GR, vol. 25, no. 6A, 1 November 2005 (2005-11-01), pages 3725-3732, XP009077768, ISSN: 0250-7005
- MORETTA A ET AL: "ACTIVATING RECEPTORS AND CORECEPTORS INVOLVED IN HUMAN NATURAL KILLER CELL-MEDIATED CYTOLYSIS", ANNUAL REVIEW OF IMMUNOL, ANNUAL REVIEWS INC, US, vol. 19, 1 January 2001 (2001-01-01), pages 197-223, XP008016880, ISSN: 0732-0582, DOI: 10.1146/ANNUREV.IMMUNOL.19.1.197
- PEIPP M ET AL: "Bispecific antibodies targeting cancer cells", BIOCHEMICAL SOCIETY TRANSACTIONS, PORTLAND PRESS LTD, GB, vol. 30, 1 January 2002 (2002-01-01), pages 507-511, XP002981328, ISSN: 0300-5127, DOI: 10.1042/BST0300507
- Raja Rajalingam: "Human diversity of killer cell immunoglobulin-like receptors and disease", The Korean Journal of Hematology, vol. 46, no. 4, 1 January 2011 (2011-01-01), page 216, XP055386149, ISSN: 1738-7949, DOI: 10.5045/kjh.2011.46.4.216

## Beschreibung

Die Erfindung betrifft rekombinante Antikörper gegen das humane La Protein umfassend eine Bindungseinheit, die spezifisch an eine Effektorzelle bindet, und ihre Anwendung zum Immunotargeting, insbesondere von Tumorzellen. Die erfindungsgemäßen Antikörper eignen sich zur Anwendung im Bereich der Medizin, der Pharmazie und in der biomedizinischen Forschung.

Das humane La-Protein (hLa) wurde ursprünglich als Autoantigen in Patienten mit systemischem Lupus erythematodes (SLE, Mattioli, M., Reichlin, M. 1974. Arthritis & Rheumatism; 17(4): 421-29.) und Sjögren-Syndrom (Alspaugh, M. A., Tan, E. M. 1975. J Clin Invest; 55(5): 1067-73) beschrieben und trägt die alternative Bezeichnung SS-B (Sjögren-Syndrom Antigen B).

Mit 2·10⁷ Molekülen pro humaner Zelle handelt es sich um ein abundantes Protein, das in allen Geweben zu finden ist. La hat eine funktionale Rolle im RNA Metabolismus und als RNA Chaperon, prozessiert pre-tRNA Prekursormoleküle und beeinflusst sowohl die Genauigkeit als auch die Effizienz der RNA Polymerase III Transkription *in vitro* (u. a. Gottlieb, E. et al. 1989 EMBO J; 8(3): 841-50).

Chambers et al. (1988. J Biol. Chem., 263, 18043-18051) bestimmten die Aminosäuresequenz des La Proteins und 3 antigene Epitopregionen und machten Vorhersagen über die Regionen, die in der RNA Bindung involviert sind.

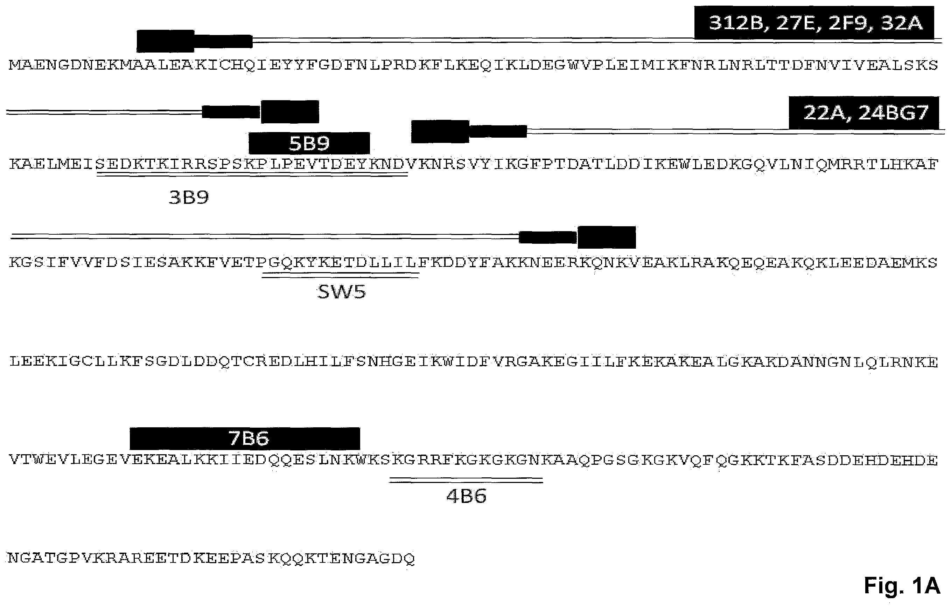

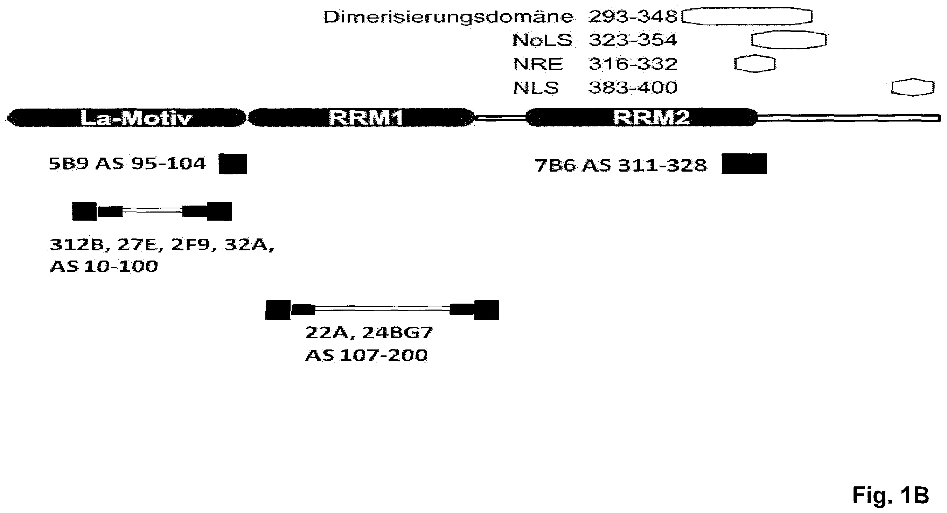

Die Primärstruktur des hLa-Proteins (Fig. 1A; SEQ ID Nr. 1) kann in drei Regionen unterteilt werden, die voneinander unabhängige räumliche Domänen bilden (s. Fig. 1B). Das La-Motiv befindet sich N-terminal, gefolgt vom zentralen RNA-Erkennungsmotiv (RNA recognition motif, RRM), das auch als RRM1 bezeichnet wird. Diese beiden Domänen bilden die N-terminale Hälfte des Proteins und werden zusammen als LaN bezeichnet. Die zweite Hälfte, LaC, enthält das C-terminale RRM (RRM2) sowie ein sich anschließendes langes, flexibles Element von etwa 80 AS, das keine Sekundärstrukturmerkmale aufweist. Nach Chambers et al. (1988) ist das N-terminale RRM1 besonders immunogen.

McNeilage et al. (1984. J. Clin. Lab. Immunol. 15, 1-17., Clin. exp. Immunol. 1985 62, 685-695.) untersuchen Anti-La-Antikörper, die durch Patienten mit Autoimmunkrankheiten gebildet werden, und welche RNA- und Protein-Komponenten von diesen Antikörpern erkannt werden. Scofield RH et al. untersuchten die Feinspezifität der Autoimmunantwort gegen die Ro/SSA and La/SSB Ribonukleoproteine (1999. Arthritis Rheum. 42(2):199-209).

Chan EK et al. (1987 J Exp Med. 166(6):1627-40) vergleichen La-Epitope, welche von humanen Autoantikörpern und Maus-Antikörpern gegen humanes und bovines La erkannt werden. Dazu wurden 5 monoklonale Maus-Antikörper hergestellt, die durch Immunisierung von Mäusen mit bovinem La erhalten wurden. Eine Kreuzreaktvität der Maus-Antikörper mit murinem La-Protein wurde nicht festgestellt.

Bachmann et al. (1990. Exp. Cell Res. 191, 171-180. 1991. Autoimmunity 9, 99-107. 1992 Autoimmunity 12, 37-45) beschreiben, dass das Autoantigen La in UV-bestrahlten Keranozyten und Herpes simplex Typ 1-infizierten Zellen an die Zelloberfläche gelangt.

US 5,457,029 beschreibt einen diagnostischen Test zum Nachweis von anti-La Antikörpern.

US 4,751,181 offenbart ein Verfahren zur Herstellung des La-Proteinantigens.

US 4,784,942 offenbart einen murinen anti-La Antikörper (Hybridoma LA1, ATCC: HB-8609), welcher durch Immunisierung mit bovinem La-Protein erhalten wurde.

Smith PR et al. (1985 J Immunol Methods. 77(1):63-76) beschreiben einen murinen anti-La Antikörper (SW5), welcher durch Immunisierung mit La-Protein aus Kaninchen erhalten wurde.

Humane monoklonale anti-La-Antikörper wurden durch Mamula MJ (1989 J Immunol 143(9):2923-8) beschrieben.

Offen D et al. (1990 J Autoimmun. 3(6):701-13) beschreiben murine anti-La Antikörper, die gegen einen in SLE-Patienten verbreiten Idiotyp (16/6 Id) gerichtet sind.

Nachfolgend werden die aus dem Stand der Technik bekannten Antikörper gegen das humane La-Protein zusammengefasst:
- LalB5 Bachmann et al. PNAS USA Vol. 83: 7770-7774, 1986;
- 8G3 und 9A5 Mamula et al. J Immunol 143(9):2923-2928, 1989;
- anti human La Carmo-Fonseca et al. ExpCellRes 185(1):73-85, 1989;
- 4B6 Tröster et al. J Autoimmunity 8(6): 825-842, 1995;
- SW1, SW3, SW5 Smith PR et al. J Immunol Methods. 77(1): 63-76, 1985; Pruijn et al. Eur J Biochem 232: 611-619, 1995;
- 3B9 Tran et al. Arthritis Rheum 46(1): 202-208, 2002;
- DAB4 Al Ejeh et al. Nucl Med Biol 2009 36(4): 395-402.

Bei allen dieser Antikörper handelt es sich um monoklonale Antikörper, die an das humane La-Protein binden. Die CDR-Sequenzen dieser Antikörper sind außer für SW5 unbekannt.

4B6 bindet das La-Epitop mit der Sequenz SKGRRFKGKGKGN (AS 330-343 des humanen La-Proteins, SEQ ID Nr.2).

Al-Ejeh F et al. (2007. Clin Cancer Res. 13(18 Pt 2):5509s-5518s) beschreiben, dass das La-Protein in Tumorzellen vermehrt exprimiert wird. Es wurde festgestellt, dass mit zunehmender DNA-Schädigung der anti-La Antikörper 3B9 vermehrt an Tumorzellen bindet. Es wird beschrieben, dass das La-Antigen in toten malignen Zellen durch Transglutaminase 2 kreuzvernetzt wird. Darauf aufbauend beschreiben Al-Ejeh F et al. (2007. Clin Cancer Res. 13(18 Pt 2):5519s-5527s) in einem Maus-Modell die Verwendung von anti-La 3B9 zum *in vivo* Targeting von Tumorzellen. Durch gleichzeitige Zytostatikabehandlung wurde das Targeting noch verbessert. Al-Ejeh F et al. (2009. PLoS One. 4(2):e4630) beschreiben eine Radioimmuntherapie, bei dem ein Tumortargeting mit dem monoklonalen anti-La Antikörper DAB4 erfolgt. Die Radioimmuntherapie wird in Kombination mit einer Chemotherapie durchgeführt. Auch WO 2008/043148 A1 offenbart die Kombinationstherapie von anti-La Antikörpern mit Zytostatika.

In Al-Ejeh F et al. (2009. PLoS One. 2009;4(2):e4558) wird die Verwendung des anti-La Antikörpers DAB4 zur Detektion der Tumorantwort auf DNA-schädigende Chemotherapeutika beschrieben.

Eine generelle Problematik für die therapeutische Wirksamkeit monoklonaler Antikörper bei der Tumorbehandlung ist das Bindungsvermögen der Antikörper an die Krebszellen, also die Affinität der Antikörper und die Auswahl des geeigneten Antigens, welches durch die Antikörper gebunden wird. Spezifische Tumorantigene werden meist nicht in ausreichenden Mengen auf den Krebszellen exprimiert. Bei Krebszellen, die ausreichend Tumorantigene exprimieren, ist die Bindungsrate der verwendeten Antikörper oft nicht ausreichend hoch. Mit einer Molekülmasse von etwa 150 kDa sind Antikörper außerdem generell in ihrer Gewebegängigkeit eingeschränkt. In diesem Fall haben Antikörperfragmente, wie Fab, F(ab)₂ oder scFv (single chain variable fragments), aufgrund ihrer deutlich geringeren Größe erhebliche Vorteile.

Bispezifische Antikörper, also Antikörperderivate aus Bestandteilen zweier unterschiedlicher monoklonaler Antikörper, bieten neue Möglichkeiten für Therapiekonzepte in der Krebsimmuntherapie.

US 2005/0136050 A1 offenbart bispezifische Antikörper, die an zwei unterschiedliche Targets binden können. Diese dienen zur Rekrutierung von humanen Immuneffektorzellen an ein Zielantigen, welches sich auf einer Zielzelle befindet.

Quadroma sind bispezifische Antikörper der ersten Generation und bestehen aus je einer schweren und einer leichten Kette zweier unterschiedlicher monoklonaler Antikörper. Die beiden Arme des Antikörpers sind dabei gegen jeweils andere Antigene gerichtet. Der Fc-Teil wird gemeinsam aus den beiden schweren Ketten der Antikörper gebildet. Durch diesen Aufbau ist es beispielsweise möglich das Paratop eines gegen ein Tumorantigen gerichteten Antikörpers und das Paratop eines anderen gegen ein Lymphozyten-Antigen gerichteten Antikörpers, auf je einem Arm des bispezifischen Antikörpers zu platzieren. Es ist so möglich, einen Drei-Zell-Komplex zu bilden, der aus den jeweils durch die unterschiedlichen Paratope gebundenen Zellen und der an den Fc-Teil gebundenen Effektorzelle ergibt. Dabei ergibt sich im Regelfall eine verbesserte Aktivierung der körpereigenen Immunzellen gegenüber den Tumorzellen.

Bispezifische Antikörper der neueren Generation sind aus zwei unterschiedlichen scFv-Fragmenten aufgebaut. Diese sind untereinander mit einem Linkerpeptid verbunden. So kann ein bispezifischer Antikörper beispielsweise mit einem scFv an Tumorzellen und mit dem anderen scFv an Effektorzellen binden.

Wird ein Paratop gegen T-Zellen gerichtet, so können auch diese Zellen aktiviert werden. Mit normalen monoklonalen Antikörpern ist dies nicht möglich, da T-Zellen über keine Fc-Rezeptoren verfügen. Bispezifische Antikörper weisen darüber hinaus ein höheres zytotoxisches Potenzial auf. Sie binden auch an Antigene, die relativ schwach exprimiert werden.

Bis heute sind noch keine bispezifischen Antikörper für die klinische Anwendung beim Menschen zugelassen.

Es sind bispezifische Antikörper bekannt, bei denen ein scFv an den CD3-Komplex auf T-Zellen bindet, diese werden auch als BiTE bezeichnet (bispecific T-cell engager) (P. A. Baeuerle et. al., BiTE: Teaching antibodies to engage T cells for cancer therapy. Curr Opin Mol Ther 11, 2009, S. 22-30).

Momentan befinden sich zwei unterschiedliche BiTE-Antikörper in klinischen Studien. Blinatumomab, ein Antikörper der gegen CD3 und CD 19 gerichtet ist, wird bei Patienten in späten Phasen des Non-Hodgkin-Lymphoms und bei Patienten mit akuter lymphoblastischer Leukämie der B-Zellreihe (B-ALL) getestet. MT110 ist ein Antikörper, der gegen CD3 und EpCAM (epithelial cell adhesion molecule) gerichtet ist und wird bei Patienten mit Bronchialkarzinom und Patienten mit gastrointestinalen Krebserkrankungen getestet (R. Bargou en. al., Tumor regression in cancer patients by very low doses of a T cell-engaging antibody. Science 321, 2008, S. 974-977; K. Brischwein et.al., MT110: a novel bispecific single-chain antibody construct with high efficacy in eradicating established tumors. Mol Immunol 43, 2006, S. 1129-1143).

Nicht alle monoklonalen Antikörper eignen sich in der Form von scFv-Fragmenten oder für die Konstruktion von bispezifischen Konstrukten. Hier ist besonders die Affinität der Antikörper entscheidend, die durch die variablen Regionen bestimmt wird. Nur besonders hochaffine Antikörper eignen sich als scFv-Fragmente, da die Bindung an das jeweilige Antigen nur mit einem Paar aus variablen Regionen der schweren und leichten Ketten erfolgt, im Gegensatz zu kompletten IgG-Antikörpern, die zwei Paare aus variablen Regionen der schweren und leichten Ketten besitzen.

Für die Behandlung von Karzinomen besteht Bedarf an neuen therapeutischen Konzepten.

Ein großes Problem beim Immuno-Targeting von Zellen, insbesondere bei der Immuntherapie von Tumoren, ist entweder das Fehlen von spezifischen Targets oder der Verlust eines spezifischen Targets bei einem Teil der Tumorzellen. Somit konnte bisher auch für viele Zielzellen kein geeignetes Targetingmodul entwickelt werden.

Aufgabe der Erfindung ist die Bereitstellung von verbesserten Antikörpern, welche universale Zielstrukturen auf der Oberfläche von Tumorzellen binden.

Die Erfindung beruht auf der Erkenntnis, dass das Kernantigen La von geschädigten bzw. sterbenden Zellen freigesetzt wird und an benachbarte intakte Zellen binden kann, wo es für mehr als 24 h auf deren Oberfläche stabil und für Antikörper zugänglich ist. Über Antikörperbindung können sogar NK Zellen aktiviert werden und somit zu einer Antikörper-vermittelten Zerstörung (ADCC) der intakten Zellen führen.

Im Rahmen der Tumortherapie (z. B. mit Zytostatika oder einer Strahlentherapie) wird das Kernantigen La von apoptotischen Tumorzellen freigesetzt und bindet an die Oberfläche von benachbarten Tumorzellen. Somit stellt das La-Protein ein induzierbares Universal-Oberflächentarget (eine universale Zielstruktur auf der Oberfläche der Tumorzellen) dar.

Ziel der Erfindung war es also, Antikörper bzw. rekombinante Antikörperderivate zu entwickeln, die es erlauben, Zielzellen (insbesondere Tumorzellen) zu erkennen und Immuneffektorzellen (z. B. T-Zellen, NK-Zellen, Dendritische Zellen) dagegen zu aktivieren.

Eine weitere Aufgabe der Erfindung ist daher die Bereitstellung von neuen rekombinanten anti-La Antikörpern, insbesondere mit einer hohen Affinität zu La, die es ermöglicht, die Antikörper als rekombinante Fragmente zum Immunotargeting zu verwenden, wobei die Antikörper eine Bindungseinheit eines Antikörpers oder eines Liganden umfassen, die spezifisch an eine Effektorzelle bindet. Erfindungsgemäß wird die Aufgabe gelöst durch neue rekombinante anti-La Antikörper welche die besagte Bindungseinheit umfassen, und welche komplementaritätsbestimmende Regionen (complementary determining regions, CDRs) enthalten, die dadurch gekennzeichnet sind, dass die CDRs der variablen Region der leichten Kette (V_{L} - linke Spalte) und die CDRs der variablen Region der schweren Kette (V_{H} - rechte Spalte) die in einer der Tabellen 1 bis 8 angegebenen Sequenzen umfassen:

**Antikörper 5B9 (Tabelle 1):**

| | V_{L} | SEQ ID Nr. | V_{H} | SEQ ID Nr. |
|---|---|---|---|---|
| CDR1 | KSSQSLLNSRTPKNYLA | 3 | HYYIY | 4 |
| CDR2 | WASTRKS | 5 | GVNPSNGGTHFNEKFKS | 6 |
| CDR3 | KQSYNLLT | 7 | SEYDYGLGFAY | 8 |

**Antikörper 7B6 (Tabelle 2):**

| | V_{L} | SEQ ID Nr. | V_{H} | SEQ ID Nr. |
|---|---|---|---|---|
| CDR1 | RSSQSLLDSRTRKNYLA | 9 | DFWMN | 10 |
| CDR2 | WASTRES | 11 | QIRNKPNNYETYYSDSLKG | 12 |
| CDR3 | KQSYNLPT | 13 | LGNSWFAY | 14 |

**Antikörper 22A (Tabelle 3):**

| | V_{L} | SEQ ID Nr. | V_{H} | SEQ ID Nr. |
|---|---|---|---|---|
| CDR1 | SASSSVSYMY | 15 | NYYIY | 16 |
| CDR2 | DTSKLAS | 17 | YIYPGNGGTAYNQKFKD | 18 |
| CDR3 | QQWSSNPQ | 19 | RGALGYYFDY | 20 |

**Antikörper 24BG7 (Tabelle 4):**

| | V_{L} | SEQ ID Nr. | V_{H} | SEQ ID Nr. |
|---|---|---|---|---|
| CDR1 | SASSSVTYMH | 21 | NYGIS | 22 |
| CDR2 | DTSKLAS | 23 | EIYRGSGNSYYNEKFKG | 24 |
| CDR3 | QQWISNPPT | 25 | GGLSFAY | 26 |

**Antikörper 312B (Tabelle 5):**

| | V_{L} | SEQ ID Nr. | V_{H} | SEQ ID Nr. |
|---|---|---|---|---|
| CDR1 | RASENIYTYLA | 27 | DYWIE | 28 |
| CDR2 | NAKTLAE | 29 | EILPGSVSIKYNEKFKG | 30 |
| CDR3 | QHHYGTPYT | 31 | SRSIYDGYFYY | 32 |

**Antikörper 27E (Tabelle 6):**

| | V_{L} | SEQ ID Nr. | V_{H} | SEQ ID Nr. |
|---|---|---|---|---|
| CDR1 | SASSSVSYMY | 33 | SYGIN | 34 |
| CDR2 | RTSNLAS | 35 | EIYPGSGTTFYNEKFRG | 36 |
| CDR3 | QQYHSYPRT | 37 | HGGYPFYFDY | 38 |

**Antikörper 2F9 (Tabelle 7):**

| | V_{L} | SEQ ID Nr. | V_{H} | SEQ ID Nr. |
|---|---|---|---|---|
| CDR1 | RASESVDSYGNSFMH | 39 | TSGMGVS | 40 |
| CDR2 | RASNLES | 41 | HIYWDDDKGYNPSLKS | 42 |
| CDR3 | QQSNEDPPT | 43 | GDVEFDY | 44 |

**Antikörper 32A (Tabelle 8):**

| | V_{L} | SEQ ID Nr. | V_{H} | SEQ ID Nr. |
|---|---|---|---|---|
| CDR1 | SASSSVSYMY | 45 | TYGLT | 46 |
| CDR2 | RTSNLAS | 47 | EIFPGSGTTFYNEKFND | 48 |
| CDR3 | QQYHSYPRT | 49 | YSNYPYYFDY | 50 |

Die Antikörper weisen bevorzugt folgende ggf. humanisierte variable Regionen der leichten Ketten (V_{L}) und/oder schweren Ketten auf (V_{H}) auf:

**Tabelle 9:**

| Antikörper | V_{L} SEQ ID Nr. | V_{H} SEQ ID Nr. |
|---|---|---|
| 5B9 | 51 | 52 |
| 7B6 | 53 | 54 |
| 22A | 55 | 56 |
| 24BG7 | 57 | 58 |
| 312B | 59 | 60 |
| 27E | 61 | 62 |
| 2F9 | 63 | 64 |
| 32A | 65 | 66 |

Die genannten anti-La Antikörper, hierin auch bezeichnet als "anti-La", sind durch Ihre CDRs und bevorzugt die oben genannten Aminosäuresequenzen der variablen Regionen der schweren und der leichten Ketten, charakterisiert. Weiterhin offenbart sind auch Gensequenzen, welche für die oben genannten Aminosäuresequenzen der variablen Regionen der leichten Ketten (V_{L}) und/oder schweren Ketten auf (V_{H}) codieren:

**Tabelle 10:**

| Antikörper | V_{L}-DNA SEQ ID Nr. | V_{H}-DNA SEQ ID Nr. |
|---|---|---|
| 5B9 | 67 | 68 |
| 7B6 | 69 | 70 |
| 22A | 71 | 72 |
| 24BG7 | 73 | 74 |
| 312B | 75 | 76 |
| 27E | 77 | 78 |
| 2F9 | 79 | 80 |
| 32A | 81 | 82 |

Vorteilhaft können die anti-La Antikörper das La-Epitop auch dann noch binden, wenn das Kernantigen auf der Oberfläche von Zellen gebunden vorliegt. In einem Zellkulturmodell wurde gezeigt, dass die erfindungsgemäßen anti-La Antikörper an die Zelloberfläche gebundenes La-Protein binden und die Zellen in einer antikörperabhängig zellvermittelten Zytotoxizität (antibody dependent cellular cytotoxicity - ADCC) durch NK-Zellen abgetötet werden.

Die von den Antikörpern erkannten Epitope auf dem humanen La-Protein werden in nachfolgender Tabelle 11 zusammengefasst:

**Tabelle 11:**

| Antikörper | Struktur des Epitops | Epitop (AS des hLa) | Redoxabhängigkeit |
|---|---|---|---|
| 5B9 | Linear, SEQ ID Nr. 83 | 95-104 | (-) |
| 7B6 | Linear, SEQ ID Nr. 84 | 311-328 | (+) oxidiert |
| 22A | Konformation | 107-200 | (-) |
| 24BG7 | Konformation | 107-200 | (-) |
| 312B | Konformation | 10-100 | (+) reduziert |
| 27E | Konformation | 10-100 | (+) reduziert |
| 2F9 | Konformation | 10-100 | (+) reduziert |
| 32A | Konformation | 10-100 | (+) reduziert |

Offenbart ist der Antikörper 7B6 bzw. ein Antikörper mit den oben genannten CDRs der variablen Regionen der leichten Ketten (V_{L}) und schweren Ketten (V_{H}) (SEQ ID Nr. 9 bis 14) und bevorzugt den variablen Regionen der leichten Ketten (V_{L}) und/oder schweren Ketten (V_{H}) gemäß SEQ ID Nr. 53 und 54 oder entsprechenden humanisierten Strukturen. Vorteilhaft bindet dieser Antikörper ein redoxabhängiges Epitop, welches unter oxidativen Bedingungen im La-Protein zugänglich wird. Derartige oxidative Bedingungen ("oxidativer Stress") existieren in Tumorgewebe, insbesondere als Reaktion auf eine Strahlen- und/oder Chemotherapie (insbesondere Zytostatikatherapie). Als oxidativen Stress bezeichnet man eine Stoffwechsellage, bei der reaktive Sauerstoffverbindungen (ROS - reactive oxygen spezies) gebildet werden. Die Tatsache, dass das 7B6 Epitop unter diesen oxidativen Bedingungen in La zugänglich wird, ermöglicht eine spezifischere und nebenwirkungsärmere Antitumortherapie. La, das in anderem Gewebe (Nichttumorgewebe) aus sterbenden Zellen unter nicht-oxidativen Bedingungen freigesetzt wird, wird von diesem Antikörper nicht gebunden. Das Epitop von 7B6 wird erst durch oxidativen Stress zugänglich gemacht. Ansonsten ist das Epitop kryptisch und der Antikörper kann nicht an das La-Protein binden.

Weiter offenbarte Anti-La-Antikörper sind 312B, 27E, 2F9, 32A bzw. Antikörper mit den CDRs der variablen Regionen der leichten Ketten (V_{L}) und/oder schweren Ketten (V_{H}) ausgewählt aus den SEQ ID Nr. 27 bis 50 (bevorzugt in den in den Tabellen 5 bis 8 genannten Kombinationen). Diese binden an gesplittete, konformationsabhängige Epitope im humanen La-Protein, welche die Bereiche der Aminosäuresequenzen von Aminosäure 10-20 und Aminosäure 94-100 (in der oben eingefügten Tabelle und nachfolgend auch "10-100") des humanen La-Proteins umfassen. Wie die Erfinder herausgefunden haben sind diese Epitope oxidationsempfindlich.

Diese Anti-La-Antikörper erkennen ausschließlich die reduzierte Form (d. h. native Form) des La-Proteins und nicht die oxidierte Form. Da in einigen Tumoren hypoxe Bedingungen vorherrschen, sind diese anti-La Antikörper für solche hypoxe Tumore besonders geeignet, insbesondere wenn ein anti-La Targeting ohne Kombination mit Therapien (insbesondere Chemotherapie und/oder Strahlentherapie) angestrebt wird, die Sauerstoffstress und damit eine Oxidation des La Proteins bewirken. Unter hypoxen Bedingungen wird ein Sauerstoffmangel (d. h. eine Sauerstoffkonzentration, die (signifikant) niedriger ist als in gesundem Gewebe) verstanden. Dieser Sauerstoffmangel entsteht häufig in soliden Tumoren, insbesondere wenn der Tumor (bzw. Teile davon) schneller als das blutversorgende Gewebe wächst. Dies ist ein besonderer Vorteil, da hypoxe Tumoren häufig resistent gegen Chemotherapie und Strahlentherapie sind.

Ebenfalls offenbarte anti-La Antikörper sind 22A und 24BG7 bzw. Antikörper mit den CDRs der variable Regionen der leichten Ketten (V_{L}) und/oder schweren Ketten (V_{H}) ausgewählt aus den SEQ ID Nr. 15 bis 26 (bevorzugt in den in den Tabellen 3 und 4 genannten Kombinationen). Diese binden an gesplittete Epitope im humanen La-Protein, welche die Bereiche der Aminosäuresequenzen von Aminosäure 107-116 und Aminosäure 185-200 (nachfolgend auch "107-200") des humanen La-Proteins umfassen.

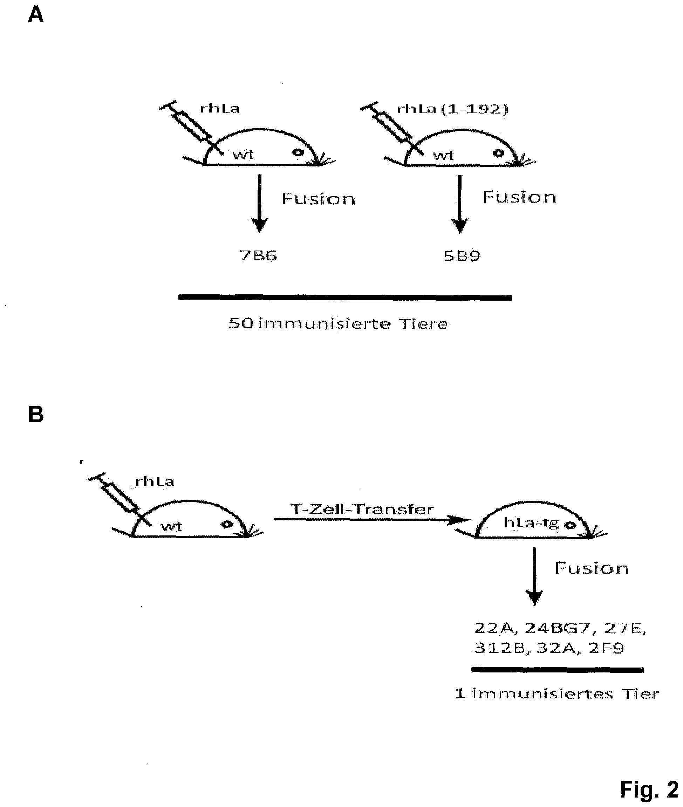

Die Antikörper wurden durch unterschiedliche Immunisierungsmethoden erhalten (s. Fig. 2). In gesunden Individuen finden keine Immunreaktionen gegen körpereigene Proteine statt, da autoreaktive T- und B-Zellen während ihrer Entwicklung eliminiert werden. Diese Toleranz und die Homologie zwischen hLa und mLa können die Ursache dafür sein, dass Mäuse nach konventioneller Immunisierung mit rekombinant hergestelltem humanen La-Protein (rhLa) kaum Antikörper gegen rhLa-Protein entwickelten. Nach einer Vielzahl von Fusionen (größer 50) konnten nur die beiden mAks 5B9 und 7B6 erhalten werden. Stattdessen wurde durch adoptiven T-Zell-Transfer eine starke B-Zell-Antwort gegen das hLa-Antigen in hLa-transgenen Mäusen induziert und aus einer Maus konnten somit mehrere mAks gewonnen werden.

In der nachfolgenden Tabelle 12 werden die Isotypen der anti-La Antikörper und die Immunisierungsmethoden zur ihrer Herstellung zusammengefasst:

**Tabelle 12:**

| Antikörper | Isotyp | Herstellung |
|---|---|---|
| 7B6 | IgG1 | Immunisierung mit rhLa-Protein |
| 5B9 | IgG2a | Immunisierung mit rhLa₁₋₁₉₂ |
| 24BG7 | IgG1 | Immunisierung von Mäusen transgen für humanes La-Protein (hLaTg), adoptiver T-Zell-Transfer |
| 22A | IgG1 | hLaTg, adoptiver T-Zell-Transfer |
| 27E | IgG2b | hLaTg, adoptiver T-Zell-Transfer |
| 312B | IgG1 | hLaTg, adoptiver T-Zell-Transfer |
| 2F9 | IgG1 | hLaTg, adoptiver T-Zell-Transfer |
| 32A | IgG1 | hLaTg, adoptiver T-Zell-Transfer |

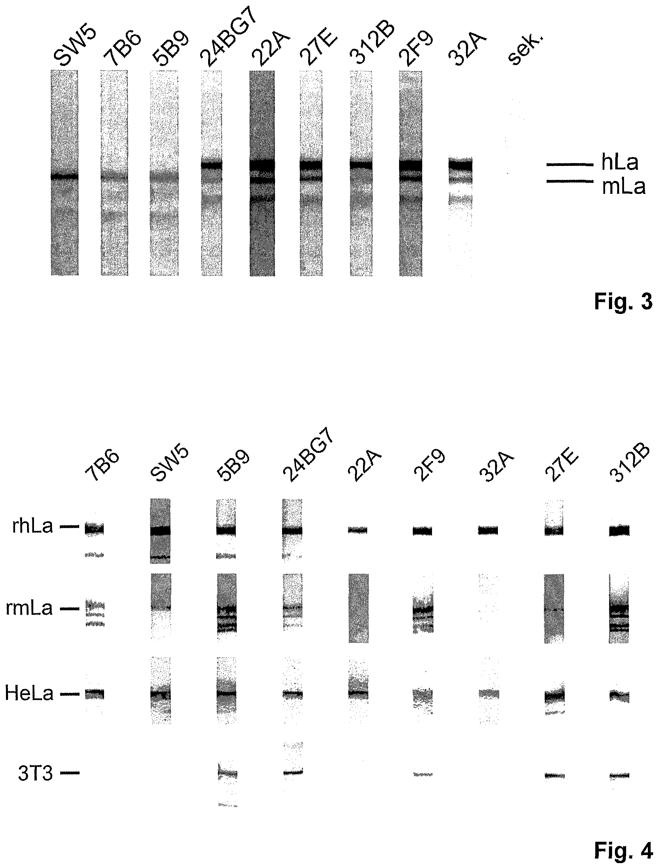

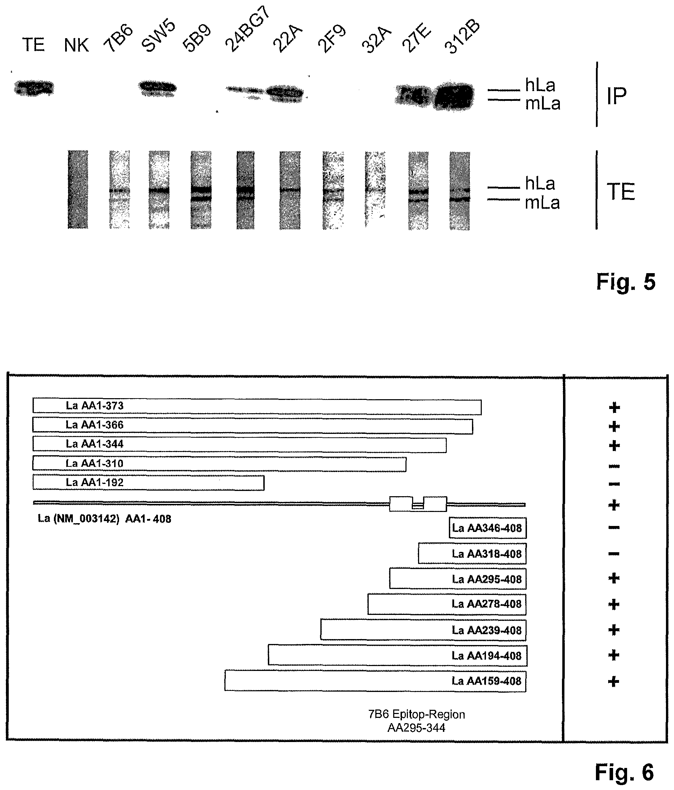

Alle genannten Antikörper sind in der Lage, rekombinant hergestelltes humanes La-Protein (rhLa, unabhängig ob prokaryont oder eukaryont hergestellt) und natives humanes La-Protein im Immunoblot (Westernblot) zu erkennen. Die Antikörper 22A und 32A sind vorteilhaft spezifisch für das humane La-Protein und können das verwandte murine La-Protein überhaupt nicht binden. Der mAk 7B6 unterscheidet zwischen prokaryont und eukaryont produzierten murinen La-Proteinen (mLa) und erkennt nur die bakterielle, wahrscheinlich nicht post-translational modifizierte Variante des murinen La-Proteins (s. Fig. 4). Die mAks 22A, 27E und 312B zeigen die stärkste Bindung an humanes La-Protein in Immunopräzipationen. Für die Präzipitation des mLa-Proteins eignete sich 312B am besten (Fig. 5).

In der nachfolgenden Tabelle 13 werden die Reaktivitäten der anti-La Antikörper im Immunoblot, in der Immunpräzipitation und der Immunfluoreszenz zusammengefasst:

**Tabelle 13:**

| mAk | Reaktivität im Immunoblot gegen | | | | Reaktivität gegen 3T3 transgen für hLa im Immunoblot | | Immunpräzipitation aus 3 T 3 L a G Totalextrakt | | Immunfluoreszenz nach Paraformaldehyd (PFA)-Fixierung | |
|---|---|---|---|---|---|---|---|---|---|---|
| | rhLa | rmLa | Humane Zellen (HeLa) | Murine Zellen (3T3) | hLa | mLa | hLa | mLa | Humane Zellen (HeLa) | Murine Zellen (3T3) |
| 7B6 | +++ | ++ | +++ | - | ++ | - | - | - | (+) | - |
| 5B9 | +++ | +++ | +++ | +++ | +++ | +++ | - | - | ++ | - |
| 24BG7 | +++ | + | +++ | + | ++ | + | + | - | ++ | ++ |
| 22A | +++ | - | +++ | - | + | - | ++ | - | ++ | - |
| 27E | +++ | + | +++ | +++ | ++ | ++ | + | + | +++ | +++ |
| 312B | +++ | +++ | +++ | +++ | + | ++ | +++ | +++ | +++ | +++ |
| 2F9 | +++ | +++ | ++ | ++ | + | + | - | - | + | + |
| 32A | +++ | - | ++ | - | (+) | - | - | - | (+) | - |

| | | | | | | | | | | |
|---|---|---|---|---|---|---|---|---|---|---|
| 3T3 LaG: murine Zellen transgen für hLa. Die Beurteilung der Bindungsstärken erfolgte nach folgender Skala: - negativ, +++ sehr stark positiv, ++ positiv, + schwach positiv, (+) sehr schwach positiv. | | | | | | | | | | |

Die Affinitäten der nativen Antikörper liegen in der Größenordnung von 10⁻¹⁰ mol/l. Die Affinitäten der rekombinanten Derivate erreichen 10⁻⁷ bis 10⁻⁹ mol/l. Durch die hohe Affinität eigenen sich die genannten CDR-Sequenzen insbesondere zur Herstellung von rekombinanten Fragmenten (wie scFv) und zum Immunotargeting.

Der Begriff "Antikörper" im erfindungsgemäßen Sinn umfasst alle Antikörper, Antikörperfragmente und Derivate derselben, die in der Lage sind, an das Antigen, in diesem Fall das humane La-Protein, zu binden und die erfindungsgemäßen CDRs komplett oder teilweise aufweisen. Dazu zählen die kompletten monoklonalen Antikörper und auch die epitopbindenden Fragmente dieser Antikörper. Hierbei umfassen die epitopbindenden Fragmente (hier auch als Antikörperfragmente oder Antikörperderivate bezeichnet) alle Teile des Antikörpers, die in der Lage sind, an das Antigen, in diesem Fall das humane La-Protein, zu binden. Beispiele für erfindungsgemäß bevorzugte Antikörperfragmente beinhalten, sind aber ausdrücklich nicht limitiert auf, Fab, Fab', F(ab')₂, Fd, Einzelketten (single-chain) variable Fragmente (scFv), Einzelketten-Antikörper, disulfidverlinkte variable Fragmente (sdFv) und Fragmente die entweder eine variable Region der leichten Kette (V_{L}) oder eine variable Region der schweren Kette (V_{H}) beinhalten. Weiterhin zählen dazu rekombinant hergestellte Antikörper, wie Diabodies, Triabodies und Tetrabodies.

Bevorzugt trägt der Antikörper ein Markermolekül, wie z. B. Biotin, Dioxygenin, ein Radionuklid oder einen Fluoreszenzfarbstoff. Besonders bevorzugt ist der Antikörper mit einer Effektorgruppe konjugiert.

Antikörperfragmente enthalten die variablen Regionen entweder allein oder in Kombination mit weiteren Regionen, die ausgewählt sind aus der Hinge-Region, und dem ersten, zweiten und dritten Bereich der konstanten Region (C_{H}1, C_{H}2, C_{H}3). Ebenfalls durch den Begriff "Antikörper" umfasst sind chimäre Antikörper, bei denen unterschiedliche Teile des Antikörpers aus verschiedenen Spezies stammen, wie beispielsweise Antikörper mit einer murinen variablen Region, welche mit einer humanen konstanten Region kombiniert ist.

Antikörperfragmente sind gegebenenfalls untereinander über ein Linkerpeptid verknüpft. Das Linkerpeptid umfasst eine kurze (bevorzugt 10 bis 20 Aminosäurereste lange), flexible Peptidsequenz, die so ausgewählt wird, dass das Antikörperfragment eine derartige dreidimensionale Faltung der V_{L} und V_{H} besitzt, dass es die Antigenspezifität des kompletten Antikörpers aufweist. Bevorzugte Linkerpeptide sind Glycin-Serin-Linker mit der Struktur (GlyₓSer)_{y} mit x und y ausgewählt aus 1 bis 10, bevorzugt 3 bis 5. Weiterhin werden Linkerpeptide bevorzugt, die aus einer Peptidsequenz bestehen, welche die Proteaseresistenz der Antikörperderivate erhöhen können. Besonders bevorzugt werden Linkerpeptide gemäß SEQ ID Nr. 85 (E7B6).

Die Bezeichnung "variable Region" ist erfindungsgemäß definiert als die Teile der schweren und leichten Ketten der Antikörper, die sich in ihrer Sequenz zwischen Antikörpern unterscheiden und die Spezifität des Antikörpers und die Bindung an sein Antigen bestimmen. Die Variabilität ist hierbei nicht gleichmäßig in der variablen Region verteilt. Sie ist üblicherweise innerhalb dreier definierter Segmente der variablen Region konzentriert, die als komplementaritätsbestimmende Regionen (CDRs) oder auch hypervariable Regionen bezeichnet werden und in den variablen Regionen sowohl der leichten als auch der schweren Ketten vorhanden sind. Die stärker konservierten Teile der variablen Regionen werden Gerüstregionen (framework regions) genannt. Die variablen Regionen der schweren und leichten Ketten enthalten vier Gerüstregionen, die überwiegend eine beta-Faltblatt Struktur annehmen, wobei jede Gerüstregion mit drei CDRs verbunden ist, die Schleifen bilden, die die beta-Faltblatt-Strukturen verbinden und in manchen Fällen Teil der beta-Faltblatt-Struktur sind. Die CDRs der jeweiligen Kette sind durch die Gerüstregionen in unmittelbare Nähe gebracht und tragen gemeinsam mit den CDRs der anderen Kette zur Bildung der Antigenbindungsregion der Antikörper bei.

Die konstante Region (Fc) der Antikörper ist nicht an der Antigenbindung beteiligt, sondern bietet stattdessen vielfältige Effektorfunktionen, die durch die Bindung an die entsprechenden Fc-bindenden Rezeptoren ausgelöst werden, wie beispielsweise die Induktion der antikörperabhängige zellvermittelte Zytotoxizität (ADCC).

Bevorzugt umfassen die erfindungsgemäßen Antikörper mindestens eine variable Region der schweren Kette (V_{H}) und eine variable Region der leichten Kette (V_{L}) in Form eines scFv. Die variable Region der schweren Kette (V_{H}) und die variable Region der leichten Kette (V_{L}) enthalten dabei jeweils mindestens eine der erfindungsgemäßen CDR-Sequenzen.

In den erfindungemäßen Antikörpern können bestimmte Aminosäuren der spezifischen Aminosäuresequenzen so ausgetauscht sein, dass sie die Bindungseigenschaften des anti-La Antikörpers beibehalten, sich jedoch in ihrer Sequenz durch Austausch, Deletion oder Addition einer oder mehrerer Aminosäuren unterscheiden. Umfasst sind somit auch Antikörper, die Strukturen enthalten, deren Aminosäuresequenzen zu den erfindungsgemäßen Aminosäuresequenzen der variablen Regionen ausgewählt aus den Sequenzen gemäß SEQ ID Nr. 51 bis 66 eine Sequenzidentität von bevorzugt mindestens 70%, besonders bevorzugt mindestens 80%, insbesondere mindestens 90% oder entsprechende humanisierte Sequenzen aufweisen und die das Antigen La binden, wobei diese Sequenzen sechs der CDRs gemäß SEQ ID Nr. 3 bis 50 in einer in der Tabelle 1 bis 8 präsentierten Kombinationen aufweisen.

In besonderen Ausgestaltungen der Erfindung umfasst der Antikörper neben den erfindungsgemäßen Aminosäuresequenzen der variablen Regionen bzw. entsprechenden humanisierten Sequenzen folgende Strukturen:
- eine konstante Region einer schweren Kette eines humanen IgG,
- eine Region C_{L} der humanen leichten Kappa-Kette
- und eine humane IgG Hinge-Region
gegebenenfalls in Form eines F(ab')₂-Fragments.

In einer besonders bevorzugten Ausgestaltung der Erfindung werden Antikörper in Form von scFv-Fragmenten welche mindestens eine variable Region der schweren Kette (V_{H}) und/oder eine variable Region der leichten Kette (V_{L}) umfassen, die die erfindungsgemäßen CDR-Regionen enthalten. Weiterhin besonders bevorzugt werden Antikörper in Form von scFv-Fragmenten welche mindestens eine der erfindungsgemäßen variablen Regionen der schweren und/oder leichten Kette umfasst.

Die Erfindung umfasst murine anti-La Antikörper und humanisierte Versionen dieser Antikörper.

Das Ziel der Humanisierung von Antikörpern liegt in der Reduktion der Immunogenität eines xenogenen Antikörpers, wie in diesem Fall des murinen Antikörpers, zur Verwendung im humanen System, wobei die volle Bindungsaffinität und die Antigenspezifität erhalten bleibt. Humanisierte Antikörper können auf verschiedenen Wegen hergestellt werden, wie beispielsweise durch Resurfacing und CDR-Grafting. Beim Resurfacing werden durch eine Kombination aus Molecular Modelling, statistischen Analysen und Mutagenese alle nicht-CDR-Regionen auf der Oberfläche des Antikörpers verändert, so dass diese der Oberfläche von Antikörpern des Zielorganismus ähneln. Beim CDR-Grafting werden die erfindungsgemäßen CDR-Regionen in humane variable Regionen eingebracht.

Humanisierte Antikörper, die die erfindungsgemäßen CDR-Regionen enthalten sind ausdrücklich Bestandteile der Erfindung.

Bestandteile der Erfindung sind auch Antikörper, die mit einer Effektorgruppe konjugiert sind. Unter Konjugation ist hierbei die Ankopplung eines Stoffs an einen Antikörper zu verstehen. Die Verbindung des Antikörpers zur Effektorgruppe wird bevorzugt durch Expression als Fusionsprotein oder durch *in vitro* Methoden hergestellt, wobei die Effektorgruppe bevorzugt über Linkergruppen an den Antikörper gebunden wird (beispielsweise über Thioetherbindungen oder Disulfidbindungen). Sie können an den Antikörper auch durch ein intermediäres Trägermolekül, beispielsweise Serumalbumin, gebunden sein. Gegebenenfalls enthält ein Antikörper auch mehrere Effektorgruppen.

Dabei sind die Effektorgruppen bevorzugt pharmazeutisch wirksame Stoffe (Wirkstoffe). Bevorzugte Wirkstoffe umfassen, sind jedoch nicht limitiert auf Toxine, wie Zytostatika, beispielsweise Maytansinoide und Maytansinoid-Analoga, Taxoide, CC-1065 und CC-1065 Analoga, Dolastatin und Dolastatin-Analoga, Methotrexat, Daunorubicin, Doxorubicin, Vincristin, Vinblastin, Melphalan, Mitomycin C, Chlorambucil und Calicheamicin. Die Erfindung umfasst auch Antikörper, welche mit Radionukliden als Effektorgruppen konjugiert sind und deren Verwendung zur Therapie und Diagnostik, insbesondere von Tumoren. Geeignete Radionuklide sind vorzugsweise die radioaktiven Isotope von Technetium, Rhenium, Yttrium, Kupfer, Gallium, Indium, Bismuth und Platin, wie z. B. ^{99m}Tc, ⁹⁰Y, ¹⁸⁶Re, ¹⁸⁸Re, ⁶⁸Ga und ¹¹¹In.

Effektorgruppen umfassen weiterhin Enzyme (besonders für das ADEPT-System geeignete Enzyme), costimulatorische Moleküle (z.B. CpG) oder auch Nukleinsäuren. Der mit einer Effektorgruppe konjugierte Antikörper kann gegebenenfalls in Form eines Fusionsproteins vorliegen.

Gegenstand der Erfindung sind rekombinante Antikörper, enthaltend:
i. eine Bindungseinheit eines Antikörpers, der spezifisch an ein Epitop eines humanen Kernantigens, bevorzugt humanes La-Protein, bindet, sowie
ii. eine Bindungseinheit
   - eines Antikörpers, der spezifisch an eine Effektorzelle bindet oder
   - eines Liganden, der spezifisch an eine Effektorzelle bindet.

Der Antikörper oder Ligand, der spezifisch an eine Effektorzelle bindet, ist bevorzugt ausgewählt aus Antikörpern und Liganden, die spezifisch Oberflächenstrukturen auf T-Lymphozyten, NK-Zellen, Dendritischen Zellen, Granulozyten und/oder Monozyten binden.

Das Epitop, welches der Antikörper, der spezifisch an ein Epitop eines humanen Kernantigens bindet, ist bevorzugt nur bei oxidierenden Bedingungen zugänglich. Ein derart bevorzugtes Antigen, welches von anti-La 7B6 erkannt wird, ist in SEQ ID Nr. 84 angegeben.

Bestandteile der Erfindung sind somit rekombinante Antikörper, die mit einem weiteren Antikörper, Antikörperfragment oder Liganden konjugiert sind, welcher gegen ein anderes Antigen als das humane Kernantigen, insbesondere das humane La-Protein, gerichtet ist. Der rekombinante Antikörper ist bevorzugt ein bispezifischer Antikörper. Bevorzugt ist der bispezifische Antikörper ein single chain bispecific diabody (scBsDb). In diesem Fall sind zwei scFv-Fragmente über einen kurzen (bevorzugt 10 bis 20 Aminosäurereste langen) Linker miteinander verbunden. Besonders bevorzugt ist der bispezifische Antikörper ein single chain bispecific tandem antibody (scBsTaFv). In diesem Fall sind die zwei scFv-Fragmente über längere (bevorzugt 18 bis 50 Aminosäurereste lange) Linkerpeptide verbunden, was in einer besonders flexiblen Struktur resultiert.

Bevorzugt enthalten die erfindungsgemäßen rekombinanten Antikörper neben dem anti-La-Antikörper einen Antikörper, Antikörperfragment oder Liganden, der spezifisch gegen Oberflächenantigene von Effektorzellen, wie zum Beispiel T Zellen, besonders zytotoxische T Zellen, NK-Zellen, Monozyten, Makrophagen, Dendritische Zellen, oder Granulozyten gerichtet ist. Die Definition von Effektorzellen im erfindungsgemäßen Sinn umfasst alle die Zellen des angeborenen und adaptiven Immunsystems, die Immunreaktionen vermitteln bzw. aktiv daran beteiligt sind. Besonders bevorzugt sind diese Antikörper gegen folgende Oberflächenstrukturen auf Effektorzellen gerichtet: CD3, CD8, CD4, CD25, CD28, CD16, NKG2D, NKp46, NKp44, aktivierende KIR-Rezeptoren (activating Killer Cell Immunoglobulin-like Receptors).

Ebenfalls Bestandteile der Erfindung sind rekombinante Antikörper, die neben dem erfindungsgemäßen anti-La-Antikörper einen Liganden umfassen, der die Aktivität von Effektorzellen durch Bindung an die Oberfläche der Effektorzellen beeinflusst. Der Ligand ist dabei so ausgewählt, dass er spezifisch an Oberflächenstrukturen von Effektorzellen bindet und durch die Bindung Signalkaskaden zur Aktivierung der Effektorzellen auslöst. Bevorzugt ist als Ligand eine Proteinstruktur oder ein Glycan, welcher spezifisch an einen Rezeptor bindet, der spezifisch auf der Oberfläche von Effektorzellen exprimiert wird, wobei der Ligand durch seine Bindung an den Rezeptor eine Aktivierung der Effektorzelle bewirkt. Besonders bevorzugt sind die Proteinstrukturen ausgewählt aus ULB-Ps (z. B. ULB-P2), MICA, MICB, sowie Zytokinen (wie z.B. IL2 und IL15) und deren Fusionsproteinen.

Die Verbindung des anti-La Antikörpers zum weiteren Antikörper, Antikörperfragment oder zur Proteinstruktur wird bevorzugt durch eine Expression als Fusionsprotein oder durch *in vitro* Methoden hergestellt, wobei die weiteren Antikörper, Antikörper oder Proteinstrukturen bevorzugt über Linker, wie Peptidlinker, an den Antikörper gebunden wird.

Die Erfindung umfasst weiterhin Nukleinsäuresequenzen, die für einen erfindungsgemäßen Antikörper kodieren, sowie Vektoren, die die Nukleinsäuresequenzen enthalten.

Der Vektor (Expressionsvektor) ist jeweils bevorzugt ein Plasmid, ein künstliches Chromosom oder auch ein Viruspartikel oder ein anderer Vektor, der eine Expressionskassette enthält, die stabil in das Genom des Wirts (Wirtszelle oder Wirtsorganismus) eingebaut wird.

Bevorzugt enthalten die Nukleinsäuresequenzen, die für einen erfindungsgemäßen Antikörper kodieren die Sequenzen, die für die oben aufgeführten CDRs (ausgewählt aus SEQ ID Nr. 3 bis 50 in den in Tabelle 1 bis 8 genannten Kombinationen) der variablen Regionen der schweren Ketten (V_{H}) und der leichten Ketten (V_{L}) kodieren.

In einer weiteren bevorzugten Ausgestaltung der Erfindung enthalten die Nukleinsäuresequenzen, Sequenzen, die für die variablen Regionen der schweren Ketten (V_{H}) und/oder der leichten Ketten (V_{L}) ausgewählt aus SEQ ID Nr. 51 bis 66 kodieren.

Bestandteile der Erfindung sind ebenfalls Wirtszellen oder nicht-menschliche Wirtsorganismen, die eine erfindungsgemäße Nukleinsäuresequenz enthalten.

Eine Wirtszelle ist eine natürlich vorkommende Zelle oder eine transformiert oder genetisch veränderte Zelllinie oder ein (multizellulärer) nicht-menschlicher Wirtsorganismus, welche bzw. welcher das erfindungsgemäße Expressionsystem (d. h. mindestens einen Expressionsvektor) enthält. Die Erfindung umfasst dabei transiente Transfektanten (z. B. durch mRNA-Injektion), also Wirte (Wirtszellen oder -organismen), in denen das Expressionsystem als Plasmid oder künstliches Chromosom enthalten ist, sowie Wirte in denen das Expressionsystem stabil in das Genom des Wirtes (oder einzelner Zellen des Wirtes) integriert ist. Die Wirtszelle ist bevorzugt ausgewählt aus Prokaryonten oder Eukaryonten. Bevorzugte Prokaryonten sind ausgewählt aus *Escherichia coli* und *Bacillus subtilis.* Bevorzugte Eukaryonten sind Hefezellen (z. B. *Saccharomyces cerevisiae, Pichia pastoris*), Insektenzellen, amphibische Zellen oder Säugetierzellen, wie z. B. CHO, HeLa, Hek293T, Hek293A. Bevorzugte Wirtsorganismen sind Pflanzen, wie z. B. Mais oder Tabak, Invertebraten oder Vertebraten, insbesondere *Bovidae, Drosophila melanogaster, Caenorhabditis elegans, Xenopus laevis, Medaka,* Zebrafisch oder *Mus musculus,* oder Zellen oder Embryonen der genannten Organismen.

Die Erfindung umfasst weiterhin eine pharmazeutische Zusammensetzung, die einen oder mehrere erfindungsgemäße Antikörper in Assoziation mit einem pharmazeutisch annehmbaren Verdünnungsmittel oder Träger enthält. Bevorzugt liegt die pharmazeutische Zusammensetzung in einer für die intravenöse Verabreichung geeigneten Form vor.

Vorzugsweise umfasst die Zusammensetzung einen chimären oder humanisierten Antikörper mit einer verringerten Immunogenität, welcher die erfindungsgemäßen CDR Regionen enthält.

Die erfindungsgemäßen pharmazeutischen Zusammensetzungen umfassen verschiedene Dosierungsformen. Die pharmazeutischen Zusammensetzungen werden bevorzugt parenteral, besonders bevorzugt intravenös verabreicht. In einer Ausgestaltung der Erfindung liegt die parenterale pharmazeutische Zusammensetzung in einer Darreichungsform vor, die zur Injektion geeignet ist. Besonders bevorzugte Zusammensetzungen sind daher Lösungen, Emulsionen oder Suspensionen des Antikörpers welches in einem pharmazeutisch annehmbaren Verdünnungsmittel oder Träger vorliegt.

Als Träger werden vorzugsweise Wasser, gepuffertes Wasser, 0,4% Salzlösung, 0,3% Glycin und ähnliche Lösungsmittel eingesetzt. Die Lösungen sind steril. Die pharmazeutischen Zusammensetzungen werden durch herkömmliche, gut bekannte Techniken sterilisiert. Die Zusammensetzungen enthalten bevorzugt pharmazeutisch akzeptable Hilfssubstanzen, wie etwa diejenigen, die erforderlich sind um näherungsweise physiologische Bedingungen zu ergeben und/oder die Stabilität des Antikörpers zu erhöhen, wie etwa Mittel zur Einstellung des pH-Werts und Puffermittel, Mittel zur Einstellung der Toxizität und dergleichen, vorzugsweise ausgewählt aus Natriumacetat, Natriumchlorid, Kaliumchlorid, Calciumchlorid und Natriumlactat. Die Konzentrationen der erfindungsgemäßen Antikörper in diesen Formulierungen sind je nach Anwendung variabel, sie betragen vorzugsweise weniger als 0,01 Gewichts-%, bevorzugt mindestens 0,1 Gewichts-%, weiter bevorzugt zwischen 1 und 5 Gewichts-% und sie werden primär auf der Basis von Fluidvolumina, Viskosität, u. ä. ausgewählt oder in Übereinstimmung mit dem jeweiligen Verabreichungsmodus.

Die erfindungsgemäßen Antikörper werden bevorzugt in eine Zusammensetzung aufgenommen, die für eine parenterale Verabreichung geeignet ist. Bevorzugt ist die pharmazeutische Zusammensetzung eine injizierbare gepufferte Lösung, die zwischen 0,1 bis 500 mg/ml Antikörper, besonders bevorzugt zwischen 0,1 bis 250 mg/ml Antikörper, insbesondere zusammen mit 1 bis 500 mmol/l (mM) Puffer enthält. Die injizierbare Lösung kann sowohl in einer flüssigen als auch ein einer lyophilisierten Dosierungsform vorliegen. Der Puffer kann bevorzugt Histidin sein (bevorzugt 1 bis 50 mM. besonders bevorzugt 5 bis 10 mM), bei einem pH-Wert von 5,0 bis 7,0 (besonders bevorzugt bei einem pH-Wert von 6,0).

Andere geeignete Puffer umfassen, sind aber ausdrücklich nicht beschränkt auf, Natriumsuccinat, Natriumcitrat, Natriumphosphat oder Kaliumphosphat. Bevorzugt wird Natriumchlorid zwischen 0 bis 300 mM, besonders bevorzugt 150 mM für eine flüssige Darreichungsform, verwendet. Für eine lyophilisierte Darreichungsform enthält die pharmazeutische Zusammensetzung bevorzugt Kälteschutzmittel, bevorzugt 0 - 10 % Sucrose (besonders bevorzugt 0,5 - 1,0 %). Andere Kälteschutzmittel umfassen Trehalose und Lactose. Für eine lyophilisierte Darreichungsform enthält die pharmazeutische Zusammensetzung bevorzugt Quellmittel, bevorzugt 1 bis 10 % Mannitol. Andere Quellmittel umfassen Glycin und Arginin. Sowohl in flüssigen als auch in lyophilisierten Darreichungsformen werden bevorzugt Stabilisatoren eingesetzt, besonders bevorzugt zwischen 1 bis 50 mM L-Methionin (besonders bevorzugt zwischen 5 und 10mM).

In einer bevorzugten Ausführungsform umfasst die pharmazeutische Zusammensetzung den Antikörper in einer Dosismenge von 0,1 mg/kg bis 10 mg/kg pro Anwendung. Besonders bevorzugte Dosismengen umfassen 1 mg/kg Körpergewicht.

Pharmazeutische Zusammensetzungen müssen steril und unter den Herstellungs- und Aufbewahrungsbedingungen stabil sein. Die Zusammensetzung kann formuliert werden als eine Lösung, Mikroemulsion, Dispersion, in Liposomen oder einer anderen geordneten Struktur, die für hohe Konzentrationen an Antikörper geeignet ist. Sterile injizierbare Lösungen können hergestellt werden, indem man den Antikörper in der erforderlichen Menge in einem geeigneten Lösungsmittel aufnimmt, mit einem oder mit einer Kombination der vorstehend aufgezählten Inhaltsstoffe je nach Bedarf, gefolgt von einer Filtersterilisation. Für sterile, lyophilisierte Pulver für die Herstellung von sterilen injizierbaren Lösungen sind die bevorzugten Herstellungsverfahren Vakuumtrocknen und Sprühtrocknen, was ein Pulver des Antikörpers plus etwaigen zusätzlichen gewünschten Inhaltsstoffen aus einer zuvor steril filtrierten Lösung davon ergibt.

Die Erfindung umfasst die Verwendung des erfindungsgemäßen Antikörpers als Arzneimittel.

Die Erfindung umfasst auch die erfindungsgemässen Antikörper zur Verwendung in einem Verfahren zur Behandlung eines Menschen der eine Tumorerkrankung aufweist, durch Verabreichung eines erfindungsgemäßen Antikörpers.

Mit Tumorerkrankungen sind insbesondere die akute myeloische Leukämie (AML), chronische myeloische Leukämie (CML) und pro-myeloische Leukämie (PML) und weitere Erkrankungen wie das Myelodysplastische Syndrom (MDS) gemeint. Neben hämatologischen Tumoren eignen sich die erfindungsgemäßen anti-La Antikörper insbesondere zur Therapie solider Tumore z.B. des Prostata-, Pankreas-, Kolon-, Lungen-, Mammakarzinoms, Karzinome der Schilddrüse, Melanome, Gehirntumore (z.B. Glioblastome) sowie Head und Neck Lymphome.

Vorteilhaft eignet sich die Erfindung insbesondere zur Immunotherapie von sogenannten "Immune escape"-Varianten von Tumoren, d. h. Tumore, welche die Expression von Tumorantigenen gestoppt bzw. herunterreguliert haben, um dem Immunsystem zu entgehen. Erfindungsgemäß wird zum Tumortargeting kein Antikörper verwendet, der gegen ein Tumorantigen gerichtet ist, sondern gegen ein Kernantigen. Durch die erfindungsgemäßen rekombinanten Antikörper, die das La-Protein (oder ein anderes Kernantigen) auf der Oberfläche von Tumorzellen, sowie Effektorzellen binden und damit zu dem Tumor rekrutieren, können so Tumorzellen mit einem Antigen an der Oberfläche getargetet werden, das in allen Zellen als Kernantigen konstitutiv exprimiert wird und nicht tumorspezifisch ist.

Besonders bevorzugt für eine Immunotherapie ist der Antikörper 7B6 bzw. ein Antikörper mit den oben genannten CDRs der variablen Regionen der leichten Ketten (V_{L}) und schweren Ketten (V_{H}) (SEQ ID Nr. 9 bis 14) und bevorzugt den variablen Regionen der leichten Ketten (V_{L}) und/oder schweren Ketten (V_{H}) gemäß SEQ ID Nr. 53 und 54 oder entsprechenden humanisierten Strukturen. Wie bereits weiter oben aufgeführt bindet dieser Antikörper ein redoxabhängiges Epitop, welches unter Bedingungen des "oxidativer Stress", wie er insbesondere in Tumorgewebe existiert freigesetzt. Die Tatsache, dass das 7B6 Epitop unter diesen oxidativen Bedingungen in La zugänglich wird, ermöglicht eine spezifischere und nebenwirkungsärmere Antitumortherapie.

Für therapeutische Anwendungen wird eine sterile pharmazeutische Zusammensetzung, enthaltend eine pharmakologisch wirksame Dosismenge eines oder mehrerer erfindungsgemäßer Antikörper einem Patienten verabreicht, um vorstehend beschriebene Erkrankungen zu behandeln.

Eine Abtötung der Tumorzellen wird bevorzugt durch die Rekrutierung von Effektorzellen erreicht. Alternativ bzw. zusätzlich durch den gezielten Transport von pharmakologischen Wirkstoffen (z.B. Toxine) und die dortige Freisetzung erzielt.

In einer besonderen Ausgestaltung der Erfindung wird ein Antikörper in Form eines bispezifischen Antikörpers verwendet. Dabei enthält der bispezifische Antikörper in einer besonders bevorzugten Ausgestaltung der Erfindung mindestens eine Bindungseinheit eines Antikörpers, der spezifisch an ein Epitop eines humanes Kernantigens, bevorzugt humanes La, bindet, sowie eine Bindungseinheit , welche gegen eine Oberflächenstruktur auf NK-Zellen gerichtet ist, bevorzugt gegen ULB-P2 (d. h. bevorzugt eine Bindungseinheit eines anti-ULB-P2 oder des ULB-P2-Liganden). Bevorzugt wird der bispezifische Antikörper zur Behandlung von AML sowie des Prostatakarzinoms verwendet.

In einer weiteren besonders bevorzugten Ausgestaltung der Erfindung enthält der bispezifische Antikörper mindestens eine Bindungseinheit eines Antikörpers, der spezifisch an ein Epitop eines humanes Kernantigens, bevorzugt humanes La, bindet, sowie eine Bindungseinheit, welche gegen eine Oberflächenstruktur auf T-Zellen gerichtet ist, bevorzugt gegen CD3 oder CD8. Bevorzugt wird der bispezifische Antikörper zur Behandlung von AML sowie des Prostatakarzinoms verwendet.

In beiden oben genannten bevorzugten Ausgestaltungen der Erfindung enthält die Bindungseinheit des Antikörpers, der spezifisch humanes La bindet, vorzugweise 6 CDRs gemäß SEQ ID Nr. 3 bis 50 in einer in der Tabelle 1 bis 8 präsentierten Kombination.

Neben dem Einsatz in der Medizin für therapeutische Zwecke eignen sich die offenbarten Antikörper für die Diagnostik, die biologische Forschung und andere Anwendungen in denen der Nachweis des La-Proteins interessant ist. Derartige Anwendungen sind insbesondere Westernblot, Immunfärbungen von Zellen (z. B. für die Durchflusszytometrie und Mikroskopie) und ELISA, sowie der Einsatz als Tracer in bildgebenden Techniken wie z.B. der CT (Computer-Tomographie), PET/CT (Positronen Emissions Tomographie). Offenbart wird auch ein Verfahren zur Herstellung eines Antikörpers mit den Schritten:
a. Immunisierung eines ersten Rezipienten mit Antigen, wobei das Antigen für den ersten Rezipienten ein Fremdantigen ist,
b. Isolation von T-Lymphozyten aus dem immunisierten Rezipienten,
c. Transfer der isolierten T-Lympozyten in einen zweiten Rezipienten, welcher das Antigen exprimiert, d.h. dass das Antigen für den zweiten Rezipienten ein Autoantigen ist,
d. Isolation von antikörperproduzierenden B-Zellen aus dem zweiten Rezipienten.

Schritt a. entspricht einer klassischen Immunisierung. Die Immunisierung kann mit oder ohne Adjuvans erfolgen, z. B. mit Peptiden oder rekombinant hergestelltem Protein.

Der erste und zweite Rezipient sind bevorzugt ausgewählt aus nicht-menschlichen Vertebraten oder nicht-menschlichen Säugetieren, insbesondere Nagetieren, wie Mäusen, Hamstern oder Ratten.

Das Antigen ist für den ersten Rezipienten ein Fremdantigen, d. h. der erste Rezipient exprimiert das Antigen nicht.

Zwischen Schritt b. und c. erfolgt bevorzugt eine Anreicherung von CD4-Zellen.

Der zweite Rezipient in Schritt c. exprimiert das Antigen, bevorzugt konstitutiv, besonders bevorzugt als Transgen. D. h. für den zweiten Rezipienten ist das Antigen ein Autoantigen. Bis auf das exprimierte Antigen ist der zweite Rezipient ansonsten möglichst genetisch identisch zum ersten Rezipient.

Nach Schritt d. erfolgt bevorzugt eine Selektion von antigenspezifischen B-Zellen, eine Hybridomaerzeugung und Screening von erzeugten Hybridoma und Antikörperproduktion nach konventionellen Methoden bzw. die Herstellung und Screening von rekombinanten Antikörper Bibliotheken.

Bevorzugt werden die variablen Regionen der leichten Kette (V_{L}) und die variable Region der schweren Kette (V_{H}) der Antigen erkennenden B-Zellen sequenziert und die CDRs bestimmt. Basierend auf diesen Sequenzdaten können vorteilhaft rekombinante Antikörperfragmente und/oder humansierte Antikörper hergestellt werden.

Die folgenden Figuren und Ausführungsbeispiele erläuterten die Erfindung näher, ohne die Erfindung auf diese zu beschränken. Die Daten zu dem monoklonalen Antikörper SW5 sind als Vergleichsbeispiel aufgeführt.

**Fig. 1A** zeigt die Aminosäuresequenz des humanen La-Proteins (SEQ ID No. 1). In der Sequenz sind die Epitope, die durch aus dem Stand der Technik bekannte Antikörper (SW5, 3B9 sowie 4B6) erkannt werden, doppelt unterstrichen. Die von den erfindungsgemäßen Antikörpern 5B9 und 7B6 erkannten Epitope sind durch schwarze Balken gekennzeichnet. Die beiden räumlichen Epitope (AS 10-100 bzw. 107-200) und die dazugehörigen anti-La mAk (312B, 27E, 2F9, 32A, bzw. 22A, 24BG7) sind ebenfalls eingezeichnet. Hervorzuheben ist, dass eine Deletion/Mutation der ersten bzw. letzten fünf Aminosäuren (schwarze breitere Balken) zu einem Verlust der Reaktivität führt.

**Fig. 1B** zeigt eine schematische Darstellung des hLa-Proteins mit seinen drei Domänen und den funktionellen Bereichen. Die verschiedenen Epitopregionen sind dargestellt und die Namen der mAks, die diese binden, sind eingezeichnet.

**Fig. 2** erläutert die verschiedenen Techniken zur Generierung der unterschiedlichen anti-La-mAks.

Fig. 2A) Die mAks 7B6 und 5B9 wurden aus Wildtyp (wt)-Mäusen erhalten, die entweder mit rekombinanten humanem La-Protein (rhLa) oder mit dem rhLa1-192-Antigen immunisiert worden waren. Fig. 2B) Die übrigen mAks stammen aus einer einzigen Maus. Diese war transgen für hLa (hLaTg) und erhielt T-Zellen von einer wt-Maus, die zuvor mit rhLa immunisiert worden war.

**Fig. 3** zeigt den Nachweis des hLa-Transgens in einem Teil der Hybridomazellen.

Von den verschiedenen Hybridomazellen wurden Totalextrakte hergestellt und auf ein 12%iges SDS-Gel aufgetragen. Nach der Elektrophorese und dem Western Blot auf eine PVDF-Membran wurden die hLa- und mLa-Proteine durch anti-La-5B9 und anti-Maus-IgG-AP (AP = alkalische Phosphatase) detektiert. Der Sekundärantikörper (sek.) allein zeigte keine Proteinbande.

**Fig. 4** zeigt die Reaktivität der verschiedenen erfindungsgemäßen mAks gegen humanes und murines La-Protein im Westernblot (Immunoblot). Auf vier getrennten 12%igen Polyacrylamidgelen wurden bakteriell hergestelltes, rekombinantes humanes bzw. murines La-Protein (rhLa bzw. rmLa) sowie Totalextrakte von humanen HeLa-Zellen (DSMZ ACC 57) bzw. von murinen 3T3-Zellen (ATCC CRL 1658) aufgetragen. Nach dem Western Blot wurde jede Membran in Streifen geschnitten, welche mit den verschiedenen Hybridomaüberständen inkubiert wurden. Die Detektion erfolgte mit anti-Maus-IgG-AP.

Alle Antikörper erkannten rhLa und eukaryontes hLa-Protein. Die mAks 22A und 32A zeigten überhaupt keine Reaktivität gegenüber rmLa und mLa, während 5B9, 24BG7, 2F9, 27E und 312B sowohl hLa als auch mLa erkennen konnten. Bei 7B6 und SW5 trat die Besonderheit auf, dass sie zwar bakteriell produziertes rmLa, nicht aber eukaryontes mLa im Immunoblot färben konnten. Ursache könnte eine post-translationale Modifikation des Proteins in den jeweiligen Epitopregionen sein, welche in 3T3-Zellen, aber nicht in Bakterien vorkommt.

**Fig. 5** zeigt die Immunpräzipitation (IP) von hLa und mLa mit den verschiedenen anti-La-mAks.

Nach Präzipitation der Immunkomplexe durch TrueBlot™ anti-Maus Ig IP Beads und Zugabe von SDS-Probenpuffer wurden die Proben auf ein 12%iges Polyacrylamidgel aufgetragen. Der SDS-PAGE folgte ein elektrophoretischer Transfer der Proteine auf eine Nitrocellulosemembran. Nach dem Blockieren der Membran wurden die La-Proteine mit 5B9 Hybridomaüberstand und Maus IgG TrueBlot™-POD detektiert. Als Positivkontrolle diente der 3T3 LaG-Totalextrakt (TE). Das unterschiedliche Laufverhalten von hLa und mLa ist als Doppelbande erkennbar. Für die Negativkontrolle (NK) wurde kein anti-La-mAk zugegeben, um den durch die *beads* erhaltenen Hintergrund zu evaluieren. Parallel wurde für alle mAks einzeln die Reaktivität gegen 3T3 LaG-Totalextrakt auf dem Immunoblot überprüft. Die Detektion erfolgte mit anti-Maus-IgG-AP.

Die Erkennung und stabile Bindung des nativen Antigens lässt sich durch Immunpräzipitation nachweisen. Die La-anti-La-Immunkomplexe können durch TrueBlot™ anti-Maus Ig IP Beads isoliert werden und anschließend kann das La-Antigen auf einem Immunoblot detektiert werden. Als Testsystem wurde ein Totalextrakt einer Maus-Zelllinie verwendet, die stabil mit hLa transfiziert war (3T3 LaG). Dadurch konnte die Bindung an beide Proteine zeitgleich untersucht werden. Zusätzlich wurde der Totalextrakt dieser Zellen auf ein weiteres SDS-Gel aufgetragen, um parallel die Reaktivität der Hybridomaüberstände gegen beide Proteine im Immunoblot zu überprüfen.

**Fig. 6 bis 8** zeigen die Charakterisierung des vom anti-La Antikörper 7B6 erkannten Peptidepitops.

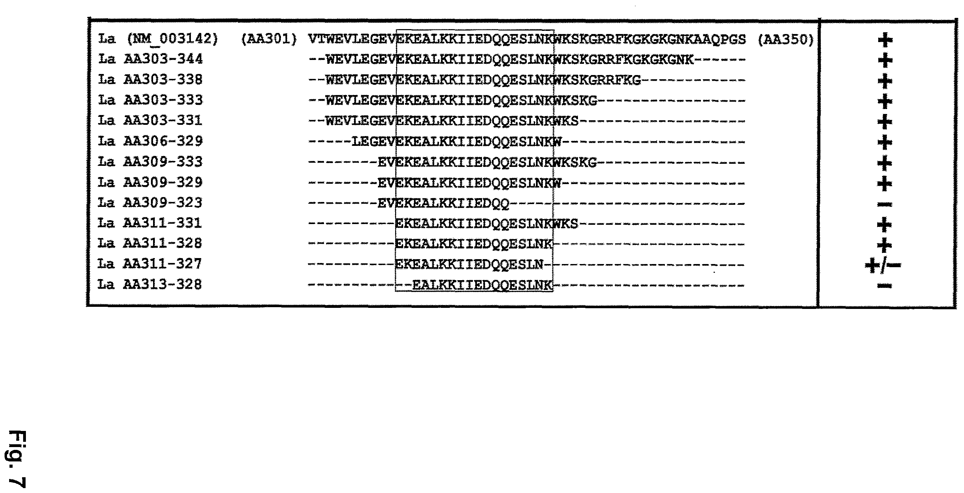

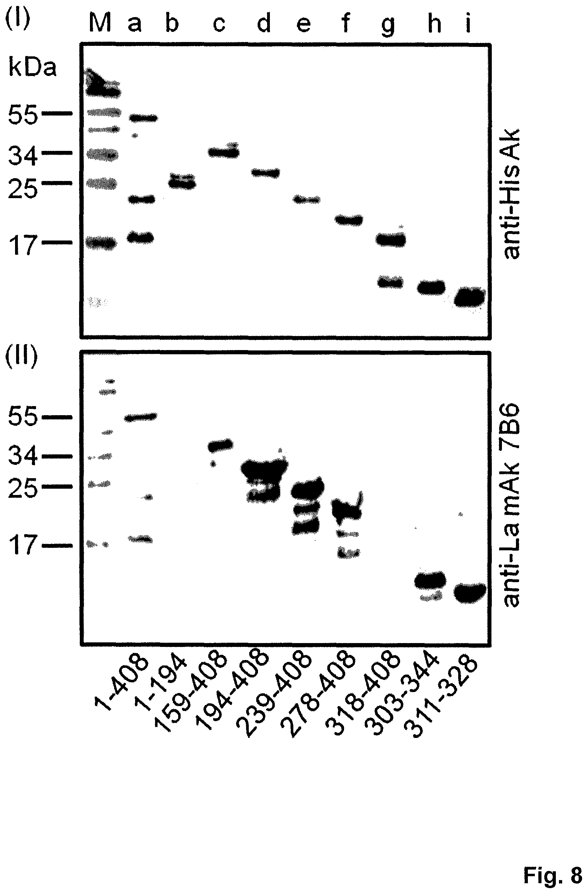

Dazu wurde eine Serie von Deletionsmutaten des La Proteins hergestellt (Fig. 6 und Fig. 7). Die gereinigten Proteine wurden durch Westernblot (Fig. 8) mit anti-penta-HIS antibody (C(I)) und dem anti-La mAk 7B6 (C(II)) analysiert. In der linken Spalte der Fig. 6 und 7 sind jeweils die Mutanten aufgeführt. Die rechte Spalte zeigt die Reaktivität im Westernblot. Die Ergebnisse zeigen, dass der Antikörper 7B6 das Epitop mit den AA311 bis 328 (SEQ ID Nr. 84) erkennt.

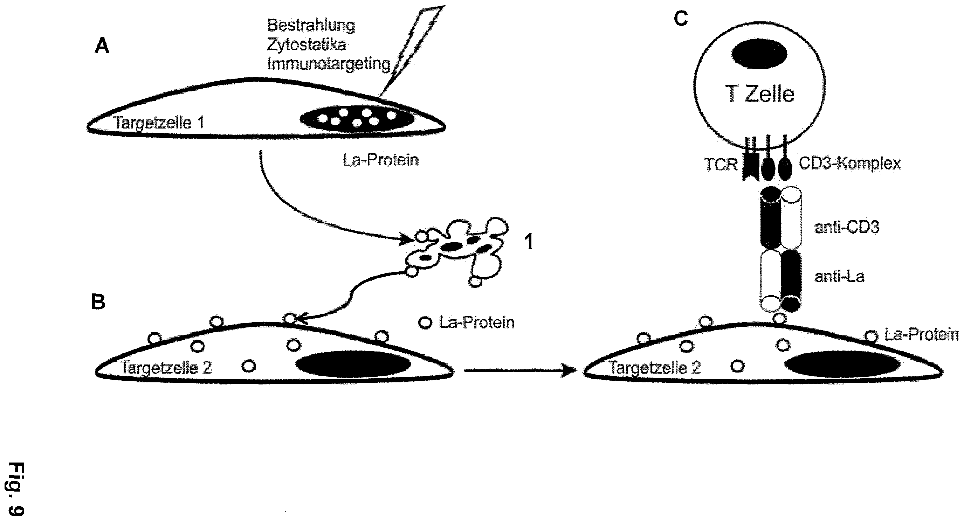

**Fig. 9** erläutert schematisch den Wirkmechanismus der Tumortherapie mit den erfindungsgemäßen Anti-La-Antikörpern.

Fig. 9A: Zellulärer Stress z.B. durch Arzneimittel (Zytostatika, Radiopharmaka), Bestrahlungen oder ein TAA-spezifisches Immuno-Targeting auf eine Zielzelle aus (Targetzelle 1) führt zur Induktion des Zelltodes der behandelten Zelle (Targetzelle 1).

Fig. 9B: Während des Zelltodes werden von der sterbenden Zelle (Targetzelle 1, 1) nukleäre Antigene (wie das La Protein) aus dem Kern freigesetzt, welche stabil an die Oberfläche der Nachbarzelle(n) (Targetzelle 2) binden.

Fig. 9C: Immuneffektorzellen (hier eine T-Zelle) werden durch ein bispezifisches Antikörper Derivat (anti-La/anti-CD3) zu der La-markierten Targetzelle 2 rekrutiert. Das bispezifische Antikörper Derivat ist einerseits gegen ein Peptidepitop im La-Protein gerichtet ist und andererseits an eine aktivierende Domäne auf der Immuneffektorzelle (hier CD3 auf T-Zellen) bindet. Durch die Bindung von La-Protein an benachbarte Zellen und die Rekrutierung von Effektorzellen zu den Targetzellen, wird es vorteilhaft möglich Tumorzellen (Targetzelle 2) zu zerstören, die ursprünglich keine spezifische Targetstruktur an der Zelloberfläche hatten. Ebenfalls können auf diese Weise z.B. Stromazellen oder Endothelzellen, die die Tumorzelle versorgen, zerstört werden.

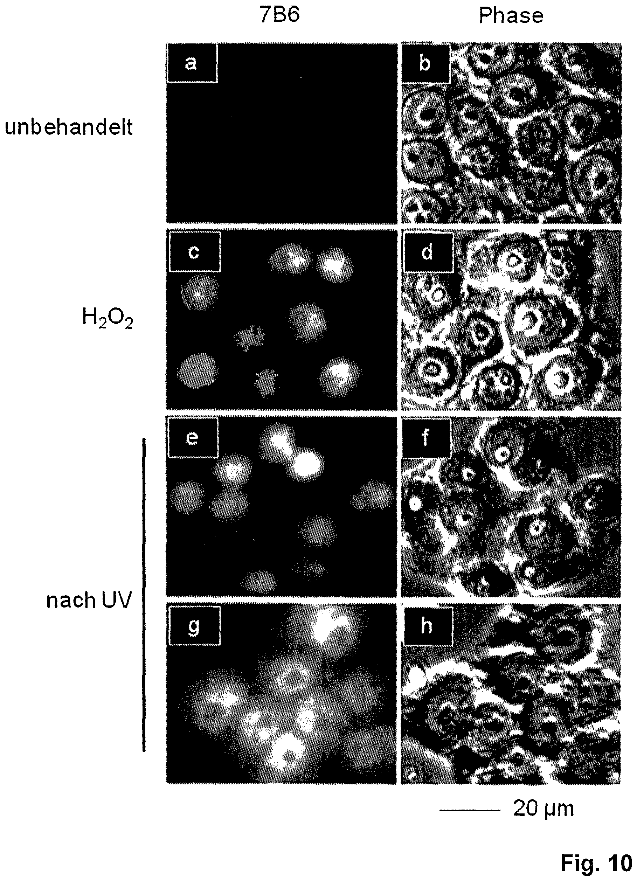

**Fig. 10** zeigt die Redoxsensitivität des La Antigens: Der Antikörper 7B6 erkennt eine oxidierte Variante. Werden humane Zellkulturzellen mit Methanol fixiert, dann lassen sich die Zellen mit dem anti-La mAk 7B6 nicht anfärben (a). Werden die Zellen vor dem Färben mit PBS, welches H₂O₂ enthält, gewaschen (c), dann kann der mAk 7B6 dagegen die Zellen anfärben. Wird vor der Fixierung oxidativer Stress (hier durch UV Strahlung) auf die Zellen ausgeübt, auch dann kann der mAk 7B6 (e) die Zellen nach der Fixierung anfärben. Dabei kommt es nach der UV-Exposition zeitabhängig zu einer Translokation in das Zytoplasma (g). (b,d,f,h) sind die korrespondierenden Phasenkontrastaufnahmen zu den Epifluoreszenzaufnahmen (a,c,e,g).

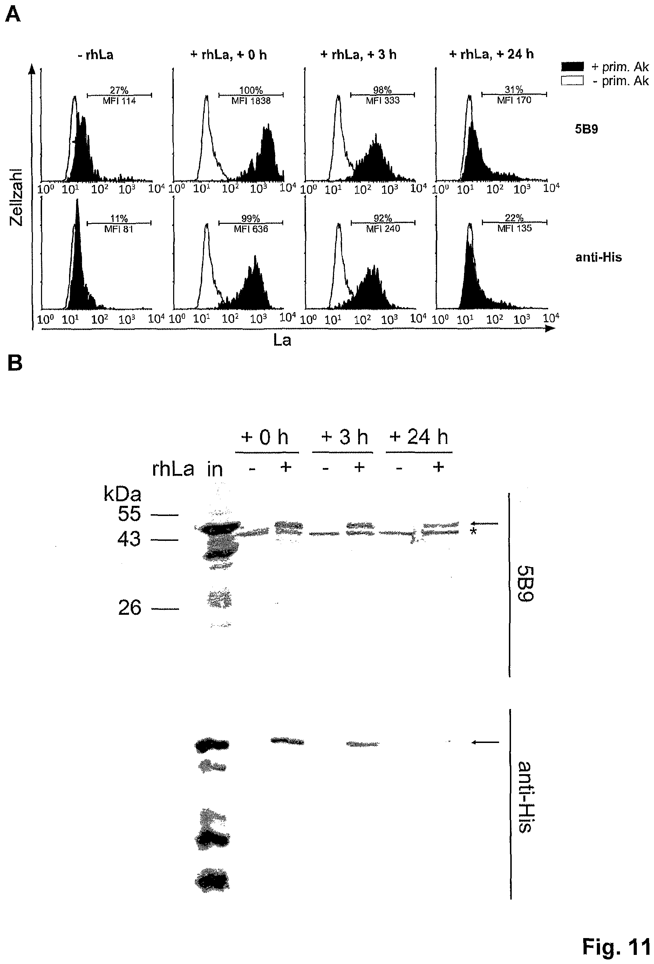

**Fig. 11** zeigt die Stabilität des rhLa-Proteins auf der Zelloberfläche von 3T3-Zellen bis zu 24 h.

Maus-3T3-Zellen wurden am Vortag in mehrere 24-well-Platten ausgesät. Alle Zellen wurden parallel für 1 h mit 1,7 µM rhLa-Protein in DMEM bei 37°C inkubiert und danach wurde das ungebundene Material weggewaschen. Zur Kontrolle wurden unbehandelte Zellen mitgeführt.
**A)** Die Zellen wurden entweder sofort (0 h) auf Eis mit 5B9 oder anti-His (prim. Ak) und dem anti-Maus-IgG-Alexa Fluor®488-Antikörper gefärbt, oder sie wurden in Medium überführt und für weitere 3 h bzw. 24 h im Brutschrank kultiviert, bevor sie ebenfalls gefärbt wurden. Nach der Färbung wurden die Zellen mit PBS-EDTA abgelöst und im Durchflusszytometer analysiert. Die länger kultivierten Zellen wurden entsprechend später gefärbt und gemessen. **B)** Western Blot der Totalextrakte, welche sofort nach La-Dekoration, nach 3 h bzw. 24 h hergestellt worden waren. Parallel wurden Proben von Zellen ohne La-Beladung mitgeführt. In der ersten Spur des Gels wurde die Menge an rhLa-Protein aufgetragen, welche auch auf die Zellen gegeben worden war ("in"). Die Western Blots wurden mit 5B9 und anti-His sowie anti-Maus-IgG-AP entwickelt. Der Pfeil markiert die Bande des rhLa-Proteins, welches mit 5B9 und anti-His detektiert werden konnte. (*) kennzeichnet das endogene mLa-Protein, welches ebenfalls von 5B9 erkannt wird.

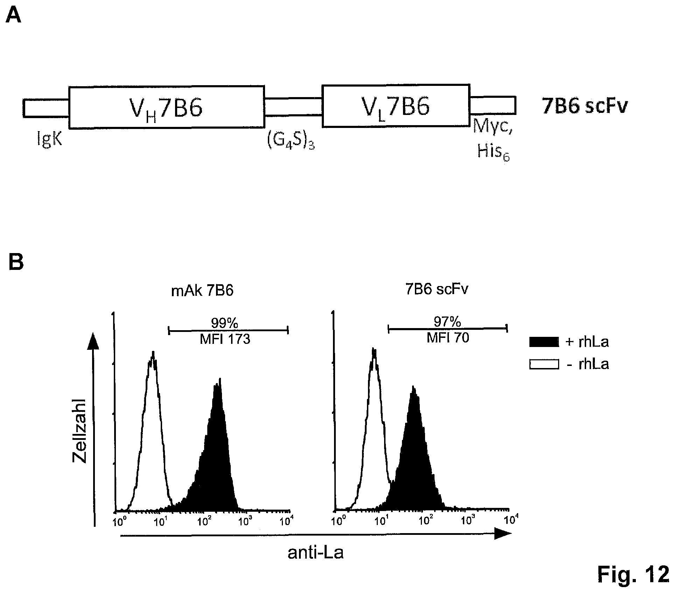

**Fig. 12** zeigt Aufbau, eukaryonte Produktion sowie Bindungsstudien für die anti-La-scFvs.
**A)** In dem anti-La-scFv-Molekül sind die variablen Domänen des mAks 7B6 in der Reihenfolge V_{H}-V_{L} über einen flexiblen Glycin-Serin-Linker ((G₄S)₃) miteinander verknüpft. Die vom Vektor pSecTag2 B codierte Igκ-Signalsequenz ermöglicht die Sekretion der Proteine in das Medium. C-terminal sind ein c-myc- und ein Hexahistidin-(His₆-) Tag angefügt. **B)** Nach der Reinigung des 7B6 scFv aus dem Zellkulturüberstand wurde die Bindung des Proteins (10 µg) auf La-dekorierten Tumorzellen untersucht. Die Detektion erfolgte mit anti-c-myc-FITC. Parallel wurden Zellen mit mAk 7B6 und anti-Maus-IgG-Alexa Fluor®488 angefärbt. Außerdem wurden jeweils Zellen ohne vorherige La-Beladung (-rhLa) mit den Antikörpern inkubiert.

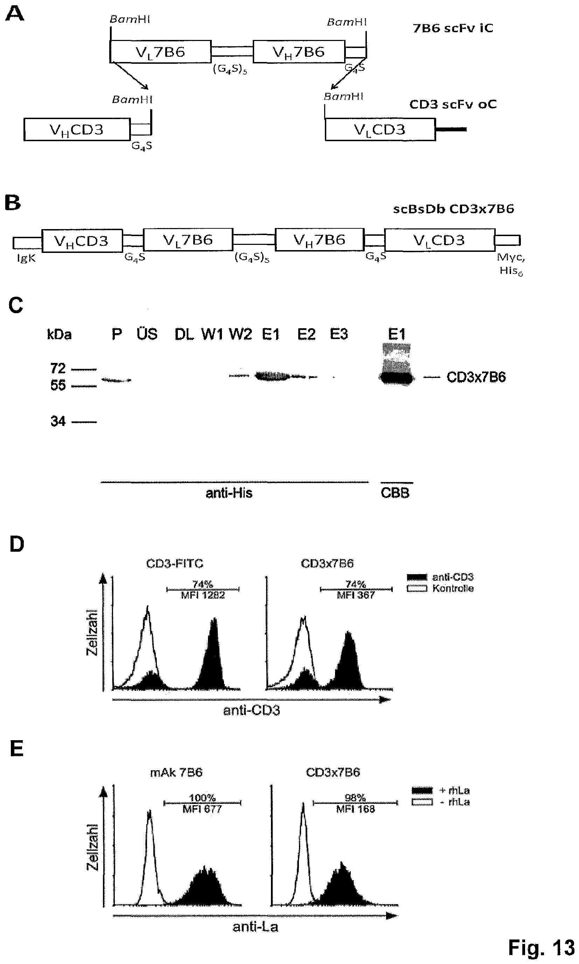

**Fig. 13** zeigt Aufbau, Reinigung und Bindungsstudien des bispezifischen Antikörpers CD3x7B6.
**A)** Die DNA-Sequenz der 7B6 scFv iC wurde über *Bam*HI-Schnittstellen in den Vektor pSecTag2 B-CD3 scFv oC kloniert, um das Plasmid pSecTag2 B-CD3x7B6 scBsDb zu erhalten. **B)** Das Protein CD3x7B6 besitzt ein N-terminales Igκ-Signalpeptid, welches die Sekretion in das Zellkulturmedium vermittelt. C-terminal sind dem Diabody ein c-myc- sowie ein Hexahistidin- (His₆-) Tag angefügt, um die Detektion des Proteins bzw. die Reinigung über Ni-NTA-Agarose zu gestatten. **C)** Reinigung des eukaryont hergestellten CD3x7B6 scBsDb. Von transduzierten HEK 293T CD3x7B6 scBsDb-Zellen wurde der Überstand (ÜS) abgenommen und auf eine Ni-NTA Spin Column aufgetragen. Der Durchlauf (DL) wurde gesammelt. Anschließend wurde mit 10 mM Imidazol (W1) und 20 mM Imidazol (W2) gewaschen. Die Elution des Diabodies erfolgte mit 350 mM Imidazol (E1-E3). Ein Teil der Zellen wurde in 2 × SDS-Probenpuffer aufgenommen (Pellet, P) und zusammen mit Aliquots aller Fraktionen auf ein 12%iges Polyacrylamidgel aufgetragen. Nach dem Western Blot auf eine Nitrocellulosemembran wurde CD3x7B6 scBsDb mit anti-His und anti-Maus-IgG-AP detektiert. Von Elution 1 wurden außerdem 10 µl auf ein zweites Gel aufgetragen, welches mit Coomassie Brillant Blau (CBB) gefärbt wurde. **D)** Bindungsanalyse auf CD3⁺ T-Lymphozyten. Als Positivkontrolle ist der Antikörper anti-CD3-FITC (schwarz) im Vergleich zu seiner Isotypkontrolle dargestellt. Parallel wurden die PBMCs mit 10 µg CD3x7B6 inkubiert, das mit anti-c-myc-FITC nachgewiesen wurde (schwarz). Als Kontrolle wurde anti-c-myc-FITC allein eingesetzt. **E)** FACS-Analyse auf HEK 293T PSCA-Zellen, welche mit 9 µM rhLa-Protein (+rhLa) oder ohne dieses (-rhLa) vorbehandelt worden waren. Die Detektion des La-Proteins erfolgte entweder mit mAk 7B6 und anti-Maus-IgG-Alexa Fluor®488 oder mit 10 µg CD3x7B6 und anti-c-myc-FITC.

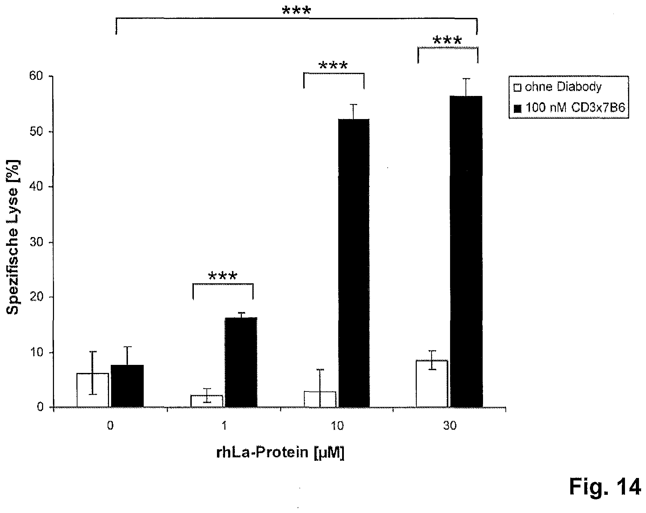

**Fig. 14** zeigt die T-Zell-vermittelte Zytotoxizität in Gegenwart von CD3x7B6.

Für den Zytotoxizitätstest wurden die Zielzellen HEK 293T PSCA mit ⁵¹Cr beladen. Danach wurde je ein Viertel der Zellen ohne La-Protein bzw. mit 1 µM, 10 µM oder 30 µM rhLa-Protein inkubiert. Anschließend wurden die Zielzellen im Verhältnis 1:20 mit voraktivierten T-Zellen kokultiviert. Des Weiteren wurde zu einem Teil der Ansätze 100 nM CD3x7B6-Protein hinzugegeben. Nach 18 h wurde die Chromfreisetzung gemessen. Dargestellt sind die Mittelwerte einer Dreifachbestimmung und deren Standardabweichungen. Die statistische Signifikanz wurde mit dem Student'schen t-Test bestimmt (***p<0,001). Von drei untersuchten ist ein repräsentativer Spender gezeigt.

### Beispiel 1 Bindung und Nachweis des rhLa-Proteins auf der Zelloberfläche

Für die Oberflächenbindungsstudien des La-Proteins wurden verschiedene Zelllinien sowie PBMCs untersucht. Die adhärenten Zelllinien wurden in 12-*well*-Platten ausgesät und am Folgetag mit 2-10 µM rhLa-Protein in PBS oder DMEM inkubiert. Die einstündige La-Bindung fand entweder auf Eis oder bei 37 °C statt. Anschließend wurden die Zellen auf Eis mit den anti-La-mAks und einem entsprechenden Sekundärantikörper gefärbt, um das La-Protein auf der Zelloberfläche nachzuweisen. Danach wurden die Zellen mit PFA-, Triton X-100- und DAPI-Lösung behandelt, bevor Immunfluoreszenzaufnahmen gemacht wurden. Für die quantitativen Bindungsstudien wurden die gefärbten, unfixierten Zellen mit PBS-EDTA abgelöst und durchflusszytometrisch untersucht. Alternativ wurden für die Durchflusszytometrie die adhärenten Zellen zuerst mit PBS-EDTA abgelöst und dann in einer 96-*well*-Spitzbodenplatte mit La und den Antikörpern inkubiert.

Im Folgenden wurde überprüft, wie lange nach Dekoration das La-Protein noch auf den Zellen detektiert werden kann und ob es eventuell proteolytisch gespalten wird und nur noch partiell auf der Zelloberfläche vorliegt. Hierfür wurden Maus-3T3-Zellen für 1 h mit rhLa in DMEM bei 37 °C inkubiert. Danach erfolgte ein Mediumwechsel. Die Zellen wurden entweder sofort untersucht oder für weitere 3 h bzw. 24 h kultiviert. Zu jedem Zeitpunkt wurden Zellen auf Eis mit den verschiedenen anti-La-mAks 27E, 5B9, SW5 (Vergleichsbeispiel), 22A und 7B6 sowie anti-His und einem entsprechenden Sekundärantikörper gefärbt. Ein Teil der Zellen wurde anschließend mit PBS-EDTA von den Kulturgefäßen abgelöst und im Durchflusszytometer analysiert (s. Fig 11A). Außerdem wurden Totalextrakte der mit La-beladenen Zellen und Kontrollzellen ohne La-Protein hergestellt. Im Western Blot wurde mit den verschiedenen Antikörpern detektiert (s. Fig. 11B).

### Beispiel 2 Immunisierung und Herstellung von Hybridoma

Für die Hybridomaherstellung wurden Mäuse (Balb/c) mit rekombinant in *E. coli* BL21 DE3 pLysS hergestellten, mittels Nickel-Affinitäts-Chromatographie gereinigtem La-Protein (rhLa - SEQ ID No. 1 mit His-tag, 40 Mäuse) immunisiert. 10 weitere Mäuse wurden mit ebenfalls in *E. coli* BL21 DE3 pLysS rekombinant hergestelltem rhLa1-192-Antigen (La-Peptid mit Aminosäureresten 1 bis 192 der SEQ ID No. 1) immunisiert. Bei der ersten Immunisierung wurden 50 µg des jeweiligen Antigens in komplettem Freund'schen Adjuvans (Difco, Michigan, USA) appliziert. Bei den weiteren Immunisierungen, die jeweils im Abstand von zwei Wochen erfolgten, wurden jeweils 25 µg Antigen appliziert. Das Antigen war hierzu in inkompletten Adjuvans (Difco, Michigan, USA) resuspendiert. Vor der Hybridomafusion waren die Tiere viermal immunisiert worden. Nach Isolierung der Milzzellen wurden diese mit Myelomzellen (P3 x Ag 8.653; ATCC CRL 1580) im Verhältnis 1:1 bis 10:1 durch tropfenweise Zugabe von Polyethylenglykol fusioniert. Anschließend wurden die Zellen unter HAT-Medium (Medium enthaltend Hypoxanthin, Aminopterin und Thymidin) selektioniert. Positive Hybridome wurden mittels ELISA identifiziert und die Zellen durch limitierte Verdünnung mehrfach rekloniert.

Im Falle der Hybridomafusion von hLa-transgenen Mäusen nach adoptivem Transfer von T-Zellen war das Protokoll folgendermaßen modifiziert. Nicht transgene Mäuse aus demselben Wurf wurden wie oben beschrieben mehrfach mit rhLa immunisiert. Danach wurden die Milzzellen präpariert und über Nylonwolle die nicht adhärenten Zellen isoliert. Die kontaminierenden B-Zellen wurden über anti-B220 magnetische beads (RA3-6B2, Dynal, Oslo, Norwegen) entfernt. Die Reinheit der isolierten Zellen wurde mittels FACS bestimmt. Im Beispiel waren 64% der isolierten Zellen CD4 positiv, 0.76% B220-positiv. Der Rest waren CD8-positive Zellen. 1x 10⁷ dieser Zellen wurden iv in die Schwanzvene am Tag Null einer 10 Wochen alten hLa transgenen Maus (A/J background) appliziert. 21 Tage nach dem adoptiven Transfer wurde die Milz entnommen und die Hybridomafusion wie oben beschrieben durchgeführt. Mittels ELISA war zuvor bestimmt worden, dass die anti-La Antwort zwischen dem 21. und 28. Tag nach dem Transfer optimal ist. Parallel wurden T-Zellen einer nichtimmunisierten Maus in eine hLa transgene Maus transferiert. Ebenso wurde eine Hybridomafusion einer nicht immunisierten Kontrollmaus durchgeführt. Anti-La Hybridome wurden nur aus der Maus etabliert, die adoptiv T-Zellen aus der La-immunisierten Maus erhalten hatte.

### Beispiel 3 Analyse von Hybridomaüberständen

Rekombinantes His₆-hLa-Protein wurde in Beschichtungspuffer verdünnt (1-5 µg/ml). Davon wurden 100 µl in jede Vertiefung der ELISA-Platte gegeben und über Nacht bei 4 °C oder 2 h bei 37 °C inkubiert. Nach fünfmaligem Waschen der Platte mit 200 µl ELISA-Waschpuffer je Vertiefung und Waschschritt wurden die übrigen Bindungsstellen mit je 200 µl ELISA-Blockierungslösung für 1 h bei 37 °C abgesättigt. Nach erneutem Waschen wurden 100 µl Hybridomaüberstand in jede Vertiefung der Platte gegeben. Die Bindung erfolgte für 1 h bei 37 °C. Anschließend wurden die ungebundenen Antikörper durch fünfmaliges Waschen entfernt und vom verdünnten Sekundärantikörper anti-Maus-IgG-POD (1:40000 in PBS) wurden 100 µl je Vertiefung aufgetragen. Nach 1 h bei 37 °C wurden die überschüssigen Antikörper wiederum durch Waschen entfernt. In jede Vertiefung wurden 100 µl der Substratlösung pipettiert und die Platte wurde im Dunkeln inkubiert. Nachdem eine deutliche Farbentwicklung erkennbar war, wurde die Reaktion mit 50 µl Stopplösung je Vertiefung beendet. Die Quantifizierung erfolgte durch Messung der optischen Dichte bei 450 nm (Referenzfilter 620 nm) mit Hilfe eines ELISA-Platten-Lesegerätes.

### Beispiel 4 Untersuchung der redoxabhängigen Antikörperbindung

Die ELISA-Platte wurde mit 10 µg/ml rhLa über Nacht bei 4 °C beschichtet. Dem schloss sich die Oxidation (3% (v/v) H₂O₂ in PBS, 30 min, RT) oder Reduktion (2% (v/v) β-Mercaptoethanol in PBS, 30 min, RT) für einen Teil der Vertiefungen an. Nach Blockierung der gesamten Platte wurde mit Hybridomaüberständen oder Patientenseren inkubiert. Die Detektion erfolgte mit anti-Maus-IgG-POD bzw. anti-human-IgG-POD.

### Beispiel 5 Herstellung von scFv-Fragmenten und bispezifischen Antikörpern

Die Herstellung von scFv-Fragmenten erfolgte ausgehend von dem mAk 7B6. Ausgehend von den Sequenzen der V_{H}- und V_{L}-Gene, die in pGEM®-T Easy kloniert waren, wurden das scFv-Derivat in den Vektor pSecTag2 B kloniert. Das entsprechende rekombinante Protein (s. Schema in Fig. 12A) verfügt über eine N-terminale Igκ-Signalsequenz, welche die Sekretion der scFv-Moleküle in das Zellkulturmedium vermittelt und dabei proteolytisch abgespalten wird. Daran schließen sich die VH- und VL-Domänen an, die über einen flexiblen Glycin-Serin-Linker ((G₄S)₃) miteinander verbunden sind. C-terminal sind den Proteinen ein c-myc-Tag sowie ein Hexahistidintag angefügt, um die spezifische Detektion bzw. die Reinigung über Ni-NTA-Affinitätschromatographie zu ermöglichen. Diese eukaryonten Expressionsvektoren wurden in HEK 293T-Zellen transfiziert. Die Totalextrakte der Zellen sowie die Zellkulturüberstände wurden im Immunoblot auf enthaltene anti-La-scFv-Moleküle analysiert.

Das 7B6 scFv-Protein wurde über Ni-NTA-Affinitätschromatographie aus dem Zellkulturmedium gereinigt. Die einzelnen Fraktionen wurden im Western Blot, der mit anti-His entwickelt wurde, auf Vorhandensein des rekombinanten Proteins untersucht. Durch FACS-Analysen (s. Fig. 12B) wurde die Bindung an mit rhLa-dekorierte Zellen untersucht. Das La-Protein wurde sowohl vom mAk 7B6 als auch vom 7B6 scFv spezifisch gebunden. Die Kontrollzellen, welche nicht mit rhLa-Protein vorbehandelt worden waren, wurden in beiden Fällen nicht angefärbt.

Nachdem der 7B6 scFv das rhLa-Protein auf der Zelloberfläche binden konnte, wurde er für die Generierung eines bispezifischen CD3x7B6 Antikörpers (Single chain Bispecific Diabody - scBsDb) verwendet. Im Plasmid pSecTag2 B-CD3 scFv oC sind die variablen Domänen in der Reihenfolge V_{H} CD3-V_{L} CD3 organisiert und durch einen flexiblen Glycin-Serin-Linker ((G₄S)₃) miteinander verbunden. Für die zweite Antigenspezifität des Diabodies wurde in diese äußere Kassette (s. Fig. 13A, CD scFv oC) die innere Kassette 7B6 scFv iC (s. Fig. 13A, CD scFv iC)mit der Domänen-folge V_{L} 7B6-(G₄S)₅-V_{H} 7B6-G₄S eingefügt. Durch Behandlung mit dem Restriktionsenzym *Bam*HI wurde der Vektor pSecTag2 B-CD3 scFv oCc zwischen V_{H} CD3 und V_{L} CD3 linearisiert und das parallel durch *Bam*HI erzeugte DNA-Fragment der 7B6 scFv iC durch Ligation eingesetzt (s. Fig. 13A). Die erhaltenen Klone wurden durch Sequenzierung überprüft. Anschließend wurde das resultierende Plasmid pSecTag2 B-CD3x7B6 scBsDb transient in HEK 293T-Zellen transfiziert, um die Produktion und Sekretion des CD3x7B6-Diabodies zu analysieren. Durch den verwendeten Vektor verfügt der bispezifische CD3x7B6 Antikörper (scBsDb) wiederum über eine N-terminale Igκ-Signalsequenz und C-terminal folgen dem Protein ein c-myc-Tag und ein Hexahistidin-Tag (s. Fig. 13B). Um für weitere Experimente ausreichende Proteinmengen zur Verfügung zu haben, wurde eine stabile Zelllinie durch Transduktion etabliert, die kontinuierlich CD3x7B6-Protein sezernierte. Hierfür war eine Amplifikation der CD3x7B6-DNA-Sequenz über PCR nötig, um N-terminal eine *Eco*RI- und C-terminal eine *Kpn*2I-Schnittstelle anzufügen. Über diese beiden Restriktionsenzyme erfolgte schließlich die Klonierung in den retroviralen Expressionsvektor pczCFG5.1. Für die Transduktion wurden ebenfalls HEK 293T-Zellen als Zielzellen verwendet.

Sowohl die transient transfizierten als auch die stabil transduzierten HEK 293T-Zellen waren in der Lage, das CD3x7B6 scBsDb-Protein herzustellen und dieses in das Zellkulturmedium abzugeben. Aus diesem konnte es über Ni-NTA-Affinitätschromatographie gewonnen werden (s. Fig. 13C). Die Bindung des Proteins an CD3+ T-Lymphozyten wurde auf humanen PBMCs untersucht (s. Fig. 13D). Das Protein konnte ebenso wie der als Positivkontrolle verwendete Antikörper anti-CD3-FITC auf 74% der Lymphozyten nachgewiesen werden. Damit wurde die Funktionalität des CD3-Arms des Diabodies unter Beweis gestellt.

Des Weiteren wurde die Bindung des CD3x7B6-Moleküls an rhLa auf der Zelloberfläche analysiert. Die 7B6-Seite des Diabodies konnte 98% der Zellen erkennen. Dies entsprach nahezu den 100% des mAks 7B6, der durch seine bivalente Bindung erwartungsgemäß eine stärkere Verschiebung der gesamten Zellpopulation im grünen Fluoreszenzkanal hervorrief (s. Fig. 13E).

Da das CD3x7B6-Protein sowohl auf La-dekorierten Tumorzellen als auch auf CD3+ T-Zellen nachgewiesen werden konnte, kann es bei der Kokultur beider Zellpopulationen eine Quervernetzung zwischen Tumorzellen und T-Effektorzellen bewirken. Dadurch werden die zytotoxischen T-Zellen aktiviert, die daraufhin die Zielzellen lysieren (siehe Schema in Fig. 9). Um diese Effektormechanismen zu verdeutlichen, wurde ein Chromfreisetzungstest durchgeführt. Die Tumorzellen wurden nach der Chrombeladung mit unterschiedlichen Mengen an rhLa-Protein inkubiert. Anschließend wurden sie für 18 h mit voraktivierten T-Zellen kokultiviert. Bei diesen T-Zellen handelte es sich um überwiegend CD8+ zytotoxische T-Zellen, die durch Inkubation von PBMCs mit IL-2 und anti-CD3 (OKT3) gewonnen wurden. Dieses Protokoll wurde am Institut für Immunologie entwickelt und die erhaltenen T-Zellen sind ausführlich charakterisiert worden. Die Ergebnisse des Chromfreisetzungstests sind in Fig. 14 gezeigt.

Für den Zytotoxizitätstest wurden die Zielzellen HEK 293T PSCA mit ⁵¹Cr beladen. Danach wurde je ein Viertel der Zellen ohne La-Protein bzw. mit 1 µM, 10 µM oder 30 µM rhLa-Protein inkubiert. Anschließend wurden die Zielzellen im Verhältnis 1:20 mit voraktivierten T-Zellen kokultiviert. Des Weiteren wurde zu einem Teil der Ansätze 100 nM CD3x7B6-Protein hinzugegeben. Nach 18 h wurde die Chromfreisetzung gemessen. Dargestellt sind die Mittelwerte einer Dreifachbestimmung und deren Standardabweichungen. Die statistische Signifikanz wurde mit dem Student'schen t-Test bestimmt (*** p<0,001). Von drei untersuchten ist ein repräsentativer Spender gezeigt.

## Patentansprüche

1. Rekombinanter Antikörper, umfassend
a.) eine Bindungseinheit eines Antikörpers, der spezifisch an ein Epitop des humanen La-Proteins bindet, sowie
b.) eine Bindungseinheit
- eines Antikörpers, der spezifisch an eine Effektorzelle bindet oder
- eines Liganden, der spezifisch an eine Effektorzelle bindet,
wobei der Antikörper, der spezifisch an eine Effektorzelle bindet, gegen eine Oberflächenstruktur ausgewählt aus CD3, CD8, CD4, CD25, CD28, CD16, NKG2D, NKp46, NKp44 und aktivierende KIR-Rezeptoren gerichtet ist,
wobei der Ligand, der spezifisch an eine Effektorzelle bindet, ausgewählt ist aus ULB-Ps, MICA, MICB, IL2 und IL15.

2. Antikörper nach Anspruch 1, der spezifisch ein Epitop des humanen La-Proteins bindet (anti-La Antikörper), enthaltend komplementaritätsbestimmende Regionen (complementarity determining regions, CDRs), **dadurch gekennzeichnet, dass** die CDRs der variablen Region der leichten Kette (V_{L}) und der variablen Region der schweren Kette (V_{H}) die folgenden Sequenzen umfassen:
| | V_{L} | SEQ ID Nr. | V_{H} | SEQ ID Nr. |
|---|---|---|---|---|
| CDR1 | KSSQSLLNSRTPKNYLA | 3 | HYYIY | 4 |
| CDR2 | WASTRKS | 5 | GVNPSNGGTHFNEKFKS | 6 |
| CDR3 | KQSYNLLT | 7 | SEYDYGLGFAY | 8 |
oder
| | V_{L} | SEQ ID Nr. | V_{H} | SEQ ID Nr. |
|---|---|---|---|---|
| CDR1 | RSSQSLLDSRTRKNYLA | 9 | DFWMN | 10 |
| CDR2 | WASTRES | 11 | QIRNKPNNYETYYSDSLKG | 12 |
| CDR3 | KQSYNLPT | 13 | LGNSWFAY | 14 |
oder
| | V_{L} | SEQ ID Nr. | V_{H} | SEQ ID Nr. |
|---|---|---|---|---|
| CDR1 | SASSSVSYMY | 15 | NYYIY | 16 |
| CDR2 | DTSKLAS | 17 | YIYPGNGGTAYNQKFKD | 18 |
| CDR3 | QQWSSNPQ | 19 | RGALGYYFDY | 20 |
oder
| | V_{L} | SEQ ID Nr. | V_{H} | SEQ ID Nr. |
|---|---|---|---|---|
| CDR1 | SASSSVSYMH | 21 | NYGIS | 22 |
| CDR2 | DTSKLAS | 23 | EIYRGSGNSYYNEKFKG | 24 |
| CDR3 | QQWISNPPT | 25 | GGLSFAY | 26 |
oder
| | V_{L} | SEQ ID Nr. | V_{H} | SEQ ID Nr. |
|---|---|---|---|---|
| CDR1 | RASENIYTYLA | 27 | DYWIE | 28 |
| CDR2 | NAKTLAE | 29 | EILPGSVSIKYNEKFKG | 30 |
| CDR3 | QHHYGTPYT | 31 | SRSIYDGYFYY | 32 |
oder
| | V_{L} | SEQ ID Nr. | V_{H} | SEQ ID Nr. |
|---|---|---|---|---|
| CDR1 | SASSSVSYMY | 33 | SYGIN | 34 |
| CDR2 | RTSNLES | 35 | EIYPGSGTTFYNEKFRG | 36 |
| CDR3 | QQYHSYPRT | 37 | HGGYPFYFDY | 38 |
oder
| | V_{L} | SEQ ID Nr. | V_{H} | SEQ ID Nr. |
|---|---|---|---|---|
| CDR1 | RASESVDSYGNSFMH | 39 | TSGMGVS | 40 |
| CDR2 | RASNLES | 41 | HIYWDDDKGYNPSLKS | 42 |
| CDR3 | QQSNEDPPT | 43 | GDVEFDY | 44 |
oder
| | V_{L} | SEQ ID Nr. | V_{H} | SEQ ID Nr. |
|---|---|---|---|---|
| CDR1 | SASSSVSYMY | 45 | TYGLT | 46 |
| CDR2 | RTSNLES | 47 | EIFPGSGTTFYNEKFND | 48 |
| CDR3 | QQYHSYPRT | 49 | YSNYPYYFDY | 50 |

3. Antikörper nach Anspruch 1 oder 2, welcher spezifisch folgendes lineares Epitop des humanen La-Proteins erkennt: EKEALKKIIEDQQESLNK (SEQ ID Nr. 84).

4. Antikörper nach einem der Ansprüche 1 bis 3, enthaltend die folgendeStruktur:
eine variable Region der leichten Kette ausgewählt aus den Sequenzen gemäß SEQ ID Nr. 51, 53, 55, 57, 59, 61, 63, 65 und
eine variable Region der schweren Kette ausgewählt aus den Sequenzen gemäß SEQ ID Nr. 52, 54, 56, 58, 60, 62, 64, 66.

5. Antikörper nach einem der Ansprüche 1 bis 4 in humanisierter Form.

6. Antikörper nach einem der Ansprüche 1 bis 5, in Form eines scFv-Fragments, eines F(ab')₂-Fragments, Diabodies, Triabodies oder Tetrabodies.

7. Antikörper nach einem der Ansprüche 1 bis 6 zur Verwendung als Therapeutikum, insbesondere zum Tumortargeting.

8. Antikörper zur Verwendung nach Anspruch 7, **dadurch gekennzeichnet, dass** die Antikörper humanes La-Protein binden, welches sich auf der Oberfläche von Tumorzellen befindet.

9. Nukleinsäuresequenz codierend für einen Antikörper nach Anspruch 1 bis 6.

10. Vektor enthaltend eine Nukleinsäuresequenz nach Anspruch 9.

11. Wirtszelle enthaltend eine Nukleinsäuresequenz nach Anspruch 9 oder einen Vektor nach Anspruch 10.

12. Nicht-menschlicher Wirtsorganismus enthaltend eine Nukleinsäuresequenz nach Anspruch 9 oder einen Vektor nach Anspruch 10 in transienter Form oder stabil in das Genom des Wirtsorganismus oder einzelner Zellen des Wirtsorganismus integriert.

13. Pharmazeutische Zusammensetzung, enthaltend einen Antikörper nach einem der Ansprüche 1 bis 6 und ein pharmazeutisch annehmbares Verdünnungsmittel oder Träger, gegebenenfalls in einer für die intravenöse Verabreichung geeigneten Form.

## Claims

1. Recombinant antibody, comprising
a.) a binding unit of an antibody that specifically binds to an epitope of a human La protein, as well as
b.) a binding unit
- of an antibody that binds specifically to an effector cell or
- of a ligand that binds specifically to an effector cell,
whereas the antibody that binds specifically to an effector cell is direct against a surface structure selected from CD3, CD8, CD4, CD25, CD28, CD16, NKG2D, NKp46, NKp44 and activating KIR receptors,
whereas the ligand that binds specifically to an effector cell, is selected from ULB-Ps, MICA, MICB, IL2 and IL15.

2. Antibody according to claim 1, which bind specifically an epitope of the human La protein (anti-La antibody) comprising complementarity determining regions (CDRs), **characterized in that** the CDRs of the variable region of the light chain (V_{L}) and the variable region of the heavy chain (V_{H}) comprise the following sequences:
| | V_{L} | SEQ ID No. | V_{H} | SEQ ID No. |
|---|---|---|---|---|
| CDR1 | KSSQSLLNSRTPKNYLA | 3 | HYYIY | 4 |
| CDR2 | WASTRKS | 5 | GVNPSNGGTHFNEKFKS | 6 |
| CDR3 | KQSYNLLT | 7 | SEYDYGLGFAY | 8 |
or
| | V_{L} | SEQ ID No. | V_{H} | SEQ ID No. |
|---|---|---|---|---|
| CDR1 | RSSQSLLDSRTRKNYLA | 9 | DFWMN | 10 |
| CDR2 | WASTRES | 11 | QIRNKPNNYETYYSDSLKG | 12 |
| CDR3 | KQSYNLPT | 13 | LGNSWFAY | 14 |
or
| | V_{L} | SEQ ID No. | V_{H} | SEQ ID No. |
|---|---|---|---|---|
| CDR1 | SASSSVSYMY | 15 | NYYIY | 16 |
| CDR2 | DTSKLAS | 17 | YIYPGNGGTAYNQKFKD | 18 |
| CDR3 | QQWSSNPQ | 19 | RGALGYYFDY | 20 |
or
| | V_{L} | SEQ ID No. | V_{H} | SEQ ID No. |
|---|---|---|---|---|
| CDR1 | SASSSVSYMH | 21 | NYGIS | 22 |
| CDR2 | DTSKLAS | 23 | EIYRGSGNSYYNEKFKG | 24 |
| CDR3 | QQWISNPPT | 25 | GGLSFAY | 26 |
or
| | V_{L} | SEQ ID No. | V_{H} | SEQ ID No. |
|---|---|---|---|---|
| CDR1 | RASENIYTYLA | 27 | DYWIE | 28 |
| CDR2 | NAKTLAE | 29 | EILPGSVSIKYNEKFKG | 30 |
| CDR3 | QHHYGTPYT | 31 | SRSIYDGYFYY | 32 |
or
| | V_{L} | SEQ ID No. | V_{H} | SEQ ID No. |
|---|---|---|---|---|
| CDR1 | SASSSVSYMY | 33 | SYGIN | 34 |
| CDR2 | RTSNLES | 35 | EIYPGSGTTFYNEKFRG | 36 |
| CDR3 | QQYHSYPRT | 37 | HGGYPFYFDY | 38 |
or
| | V_{L} | SEQ ID No. | V_{H} | SEQ ID No. |
|---|---|---|---|---|
| CDR1 | RASESVDSYGNSFMH | 39 | TSGMGVS | 40 |
| CDR2 | RASNLES | 41 | HIYWDDDKGYNPSLKS | 42 |
| CDR3 | QQSNEDPPT | 43 | GDVEFDY | 44 |
or
| | V_{L} | SEQ ID No. | V_{H} | SEQ ID No. |
|---|---|---|---|---|
| CDR1 | SASSSVSYMY | 45 | TYGLT | 46 |
| CDR2 | RTSNLES | 47 | EIFPGSGTTFYNEKFND | 48 |
| CDR3 | QQYHSYPRT | 49 | YSNYPYYFDY | 50 |

3. Antibody according to claim 1 or 2 that specifically recognizes the following linear epitope of the human La protein: EKEALKKIIEDQQESLNK (SEQ ID No. 84).

4. Antibody according to one of the claims 1 to 3, containing the following structure:
- a variable region of the light chain selected from the sequences according to SEQ ID Nos. 51, 53, 55, 57, 59, 61, 63, 65 and
- a variable region of the heavy chain selected from the sequences according to SEQ ID Nos. 52, 54, 56, 58, 60, 62, 64, 66.

5. Antibody according to one of the claims 1 to 4 in humanized form.

6. Antibody according to one of the claims 1 to 5, in the form of a scFv fragment, of a F(ab')₂ fragment, diabodies, triabodies or tetrabodies.

7. Antibody according to claim 1 to 6 for use as a therapeutic agent, in particular for tumor targeting.

8. Antibody for use according to claim 7, **characterized in that** the antibodies bind to human La protein that is located on the surface of tumor cells.

9. Nucleic acid sequence encoding for an antibody according to claim 1 to 6.

10. Vector comprising a nucleic acid sequence according to claim 9.

11. Host cell comprising a nucleic acid sequence according to claim 9 or a vector according to claim 10.

12. Non-human host organism comprising a nucleic acid sequence according to claim 9 or a vector according to claim 10 in transient form or stably integrated into the genome of the host organism or into particular cells of the host organism.

13. Pharmaceutical composition, containing an antibody according to one of the claims 1 to 6 and a pharmaceutically acceptable diluent or carrier, if necessary in a form suitable for intravenous administration.

## Revendications

1. Anticorps recombinant comprenant :
a) un groupement de liaison d'un anticorps qui se lie spécifiquement à un épitope de la protéine La humaine, et
b) un groupement de liaison
- d'un anticorps qui se lie spécifiquement à une cellule effectrice ou
- d'un ligand qui se lie spécifiquement à une cellule effectrice,
dans lequel l'anticorps qui se lie spécifiquement à une cellule effectrice est dirigé contre une structure de surface choisi parmi CD3, CD8, CD4, CD25, CD28, CD16, NKG2D, NKp46, NKp44 et les récepteurs KIR activateurs,
dans lequel le ligand qui se lie spécifiquement à une cellule effectrice est choisie parmi ULB-Ps, MICA, MICB, IL2 et de l'IL15.

2. Anticorps selon la revendication 1, qui se lie spécifiquement à un épitope de la protéine La humaine (anticorps anti-La), comprenant des régions de détermination de complémentarité (complementarity determining regions, CDRs), **caractérisé en ce que** les CDRs de la région variable de la chaîne légère (V_{L}) et la région variable de la chaîne lourde (V_{H}) comprennent les séquences suivantes :
| | V_{L} | SEQ ID N° | V_{H} | SEQ ID N° |
|---|---|---|---|---|
| CDR1 | KSSQSLLNSRTPKNYLA | 3 | HYYIY | 4 |
| CDR2 | WASTRKS | 5 | GVNPSNGGTHFNEKFKS | 6 |
| CDR3 | KQSYNLLT | 7 | SEYDYGLGFAY | 8 |
ou
| | V_{L} | SEQ ID N° | V_{H} | SEQ ID N° |
|---|---|---|---|---|
| CDR1 | RSSQSLLDSRTRKNYLA | 9 | DFWMN | 10 |
| CDR2 | WASTRES | 11 | QIRNKPNNYETYYSDSLKG | 12 |
| CDR3 | KQSYNLPT | 13 | LGNSWFAY | 14 |
ou
| | V_{L} | SEQ ID N° | V_{H} | SEQ ID N° |
|---|---|---|---|---|
| CDR1 | SASSSVSYMY | 15 | NYYIY | 16 |
| CDR2 | DTSKLAS | 17 | YIYPGNGGTAYNQKFKD | 18 |
| CDR3 | QQWSSNPQ | 19 | RGALGYYFDY | 20 |
ou
| | V_{L} | SEQ ID N° | V_{H} | SEQ ID N° |
|---|---|---|---|---|
| CDR1 | SASSSVSYMH | 21 | NYGIS | 22 |
| CDR2 | DTSKLAS | 23 | EIYRGSGNSYYNEKFKG | 24 |
| CDR3 | QQWISNPPT | 25 | GGLSFAY | 26 |
ou
| | V_{L} | SEQ ID N° | V_{H} | SEQ ID N° |
|---|---|---|---|---|
| CDR1 | RASENIYTYLA | 27 | DYWIE | 28 |
| CDR2 | NAKTLAE | 29 | EILPGSVSIKYNEKFKG | 30 |
| CDR3 | QHHYGTPYT | 31 | SRSIYDGYFYY | 32 |
ou
| | V_{L} | SEQ ID N° | V_{H} | SEQ ID N° |
|---|---|---|---|---|
| CDR1 | SASSSVSYMY | 33 | SYGIN | 34 |
| CDR2 | RTSNLES | 35 | EIYPGSGTTFYNEKFRG | 36 |
| CDR3 | QQYHSYPRT | 37 | HGGYPFYFDY | 38 |
ou
| | V_{L} | SEQ ID N° | V_{H} | SEQ ID N° |
|---|---|---|---|---|
| CDR1 | RASESVDSYGNSFMH | 39 | TSGMGVS | 40 |
| CDR2 | RASNLES | 41 | HIYWDDDKGYNPSLKS | 42 |
| CDR3 | QQSNEDPPT | 43 | GDVEFDY | 44 |
ou
| | V_{L} | SEQ ID N° | V_{H} | SEQ ID N° |
|---|---|---|---|---|
| CDR1 | SASSSVSYMY | 45 | TYGLT | 46 |
| CDR2 | RTSNLES | 47 | EIFPGSGTTFYNEKFND | 48 |
| CDR3 | QQYHSYPRT | 49 | YSNYPYYFDY | 50 |

3. Anticorps selon la revendication 1 ou 2, lequel reconnait spécifiquement l'épitope linéaire suivant de la protéine La humaine : EKEALKKIIEDQQESLNK (SEQ ID N° 84).

4. Anticorps selon l'une quelconque des revendications 1 à 3, comprenant la structure suivante :
- une région variable de la chaîne légère choisie parmi les séquences selon SEQ ID N° : 51, 53, 55, 57, 59, 61, 63, 65 et
- une région variable de la chaîne lourde choisie parmi les séquences selon SEQ ID N° : 52, 54, 56, 58, 60, 62, 64, 66.

5. Anticorps selon l'une quelconque des revendications 1 à 4 sous une forme humanisée.

6. Anticorps selon l'une quelconque des revendications 1 à 5, sous la forme d'un fragment scFv, d'un fragment F(ab')₂⁻, de di-anticorps, tri-anticorps ou tétra-anticorps.

7. Anticorps selon l'une quelconque des revendications 1 à 6 pour une utilisation en tant qu'agent thérapeutique, en particulier pour le ciblage tumoral.

8. Anticorps pour utilisation selon la revendication 7, **caractérisé en ce que** les anticorps se lient à la protéine La humaine, qui est située sur la surface des cellules tumorales.

9. Séquence d'acides nucléiques codant pour un anticorps selon la revendication 1 à 6.

10. Vecteur comprenant une séquence d'acides nucléiques selon la revendication 9.

11. Cellule hôte comprenant une séquence d'acides nucléiques selon la revendication 9 ou un vecteur selon la revendication 10.

12. Organisme hôte non humain contenant une séquence d'acides nucléiques selon la revendication 9 ou un vecteur selon la revendication 10 sous forme transitoire ou intégré de manière stable dans le génome de l'organisme hôte ou des cellules individuelles de l'organisme hôte.

13. Composition pharmaceutique comprenant un anticorps selon l'une quelconque des revendications 1 à 6 et un diluant ou support pharmaceutiquement acceptable, le cas échéant sous une forme appropriée pour une administration par voie intraveineuse.


### SEQUENCE LISTING

<110> Technische Universität Dresden
<120> Anti-La Antikörper und ihre Anwendung zum Immunotargeting
<130> 00017P0140DEWO
<150> DE 10 2010 039 018.6-41
   <151> 2010-08-06
<160> 85
<170> PatentIn version 3.5
<210> 1
   <211> 408
   <212> PRT
   <213> Mus musculus
<400> 1
   Met Ala Glu Asn Gly Asp Asn Glu Lys Met Ala Ala Leu Glu Ala Lys
   1               5                   10                  15
   Ile Cys His Gln Ile Glu Tyr Tyr Phe Gly Asp Phe Asn Leu Pro Arg
               20                  25                  30
   Asp Lys Phe Leu Lys Glu Gln Ile Lys Leu Asp Glu Gly Trp Val Pro
           35                  40                  45
   Leu Glu Ile Met Ile Lys Phe Asn Arg Leu Asn Arg Leu Thr Thr Asp
       50                  55                  60
   Phe Asn Val Ile Val Glu Ala Leu Ser Lys Ser Lys Ala Glu Leu Met
   65                  70                  75                  80
   Glu Ile Ser Glu Asp Lys Thr Lys Ile Arg Arg Ser Pro Ser Lys Pro
                   85                  90                  95
   Leu Pro Glu Val Thr Asp Glu Tyr Lys Asn Asp Val Lys Asn Arg Ser
               100                 105                 110
   Val Tyr Ile Lys Gly Phe Pro Thr Asp Ala Thr Leu Asp Asp Ile Lys
           115                 120                 125
   Glu Trp Leu Glu Asp Lys Gly Gln Val Leu Asn Ile Gln Met Arg Arg
       130                 135                 140
   Thr Leu His Lys Ala Phe Lys Gly Ser Ile Phe Val Val Phe Asp Ser
   145                 150                 155                 160
   Ile Glu Ser Ala Lys Lys Phe Val Glu Thr Pro Gly Gln Lys Tyr Lys
                   165                 170                 175
   Glu Thr Asp Leu Leu Ile Leu Phe Lys Asp Asp Tyr Phe Ala Lys Lys
               180                 185                 190
   Asn Glu Glu Arg Lys Gln Asn Lys Val Glu Ala Lys Leu Arg Ala Lys
           195                 200                 205
   Gln Glu Gln Glu Ala Lys Gln Lys Leu Glu Glu Asp Ala Glu Met Lys
       210                 215                 220
   Ser Leu Glu Glu Lys Ile Gly Cys Leu Leu Lys Phe Ser Gly Asp Leu
   225                 230                 235                 240
   Asp Asp Gln Thr Cys Arg Glu Asp Leu His Ile Leu Phe Ser Asn His
                   245                 250                 255
   Gly Glu Ile Lys Trp Ile Asp Phe Val Arg Gly Ala Lys Glu Gly Ile
               260                 265                 270
   Ile Leu Phe Lys Glu Lys Ala Lys Glu Ala Leu Gly Lys Ala Lys Asp
           275                 280                 285
   Ala Asn Asn Gly Asn Leu Gln Leu Arg Asn Lys Glu Val Thr Trp Glu
       290                 295                 300
   Val Leu Glu Gly Glu Val Glu Lys Glu Ala Leu Lys Lys Ile Ile Glu
   305                 310                 315                 320
   Asp Gln Gln Glu Ser Leu Asn Lys Trp Lys Ser Lys Gly Arg Arg Phe
                   325                 330                 335
   Lys Gly Lys Gly Lys Gly Asn Lys Ala Ala Gln Pro Gly Ser Gly Lys
               340                 345                 350
   Gly Lys Val Gln Phe Gln Gly Lys Lys Thr Lys Phe Ala Ser Asp Asp
           355                 360                 365
   Glu His Asp Glu His Asp Glu Asn Gly Ala Thr Gly Pro Val Lys Arg
       370                 375                 380
   Ala Arg Glu Glu Thr Asp Lys Glu Glu Pro Ala Ser Lys Gln Gln Lys
   385                 390                 395                 400
   Thr Glu Asn Gly Ala Gly Asp Gln
                   405
<210> 2
   <211> 13
   <212> PRT
   <213> Mus musculus
<400> 2
   Ser Lys Gly Arg Arg Phe Lys Gly Lys Gly Lys Gly Asn
   1               5                   10
<210> 3
   <211> 17
   <212> PRT
   <213> Mus musculus
<400> 3
   Lys Ser Ser Gln Ser Leu Leu Asn Ser Arg Thr Pro Lys Asn Tyr Leu
   1               5                   10                  15
   Ala
<210> 4
   <211> 5
   <212> PRT
   <213> Mus musculus
<400> 4
   His Tyr Tyr Ile Tyr
   1               5
<210> 5
   <211> 7
   <212> PRT
   <213> Mus musculus
<400> 5
   Trp Ala Ser Thr Arg Lys Ser
   1               5
<210> 6
   <211> 17
   <212> PRT
   <213> Mus musculus
<400> 6
   Gly Val Asn Pro Ser Asn Gly Gly Thr His Phe Asn Glu Lys Phe Lys
   1               5                   10                  15
   Ser
<210> 7
   <211> 8
   <212> PRT
   <213> Mus musculus
<400> 7
   Lys Gln Ser Tyr Asn Leu Leu Thr
   1 5
<210> 8
   <211> 11
   <212> PRT
   <213> Mus musculus
<400> 8
   Ser Glu Tyr Asp Tyr Gly Leu Gly Phe Ala Tyr
   1 5 10
<210> 9
   <211> 17
   <212> PRT
   <213> Mus musculus
<400> 9
   Arg Ser Ser Gln Ser Leu Leu Asp Ser Arg Thr Arg Lys Asn Tyr Leu
   1 5 10 15
   Ala
<210> 10
   <211> 5
   <212> PRT
   <213> Mus musculus
<400> 10
   Asp Phe Trp Met Asn
   1 5
<210> 11
   <211> 7
   <212> PRT
   <213> Mus musculus
<400> 11
   Trp Ala Ser Thr Arg Glu Ser
   1 5
<210> 12
   <211> 19
   <212> PRT
   <213> Mus musculus
<400> 12
   Gln Ile Arg Asn Lys Pro Asn Asn Tyr Glu Thr Tyr Tyr Ser Asp Ser
   1 5 10 15
   Leu Lys Gly
<210> 13
   <211> 8
   <212> PRT
   <213> Mus musculus
<400> 13
   Lys Gln Ser Tyr Asn Leu Pro Thr
   1 5
<210> 14
   <211> 8
   <212> PRT
   <213> Mus musculus
<400> 14
   Leu Gly Asn Ser Trp Phe Ala Tyr
   1 5
<210> 15
   <211> 10
   <212> PRT
   <213> Mus musculus
<400> 15
   Ser Ala Ser Ser Ser Val Ser Tyr Met Tyr
   1 5 10
<210> 16
   <211> 5
   <212> PRT
   <213> Mus musculus
<400> 16
   Asn Tyr Tyr Ile Tyr
   1 5
<210> 17
   <211> 7
   <212> PRT
   <213> Mus musculus
<400> 17
   Asp Thr Ser Lys Leu Ala Ser
   1 5
<210> 18
   <211> 17
   <212> PRT
   <213> Mus musculus
<400> 18
   Tyr Ile Tyr Pro Gly Asn Gly Gly Thr Ala Tyr Asn Gln Lys Phe Lys
   1 5 10 15
   Asp
<210> 19
   <211> 8
   <212> PRT
   <213> Mus musculus
<400> 19
   Gln Gln Trp Ser Ser Asn Pro Gln
   1 5
<210> 20
   <211> 10
   <212> PRT
   <213> Mus musculus
<400> 20
   Arg Gly Ala Leu Gly Tyr Tyr Phe Asp Tyr
   1 5 10
<210> 21
   <211> 10
   <212> PRT
   <213> Mus musculus
<400> 21
   Ser Ala Ser Ser Ser Val Ser Tyr Met His
   1 5 10
<210> 22
   <211> 5
   <212> PRT
   <213> Mus musculus
<400> 22
   Asn Tyr Gly Ile Ser
   1 5
<210> 23
   <211> 7
   <212> PRT
   <213> Mus musculus
<400> 23
   Asp Thr Ser Lys Leu Ala Ser
   1 5
<210> 24
   <211> 17
   <212> PRT
   <213> Mus musculus
<400> 24
   Glu Ile Tyr Arg Gly Ser Gly Asn Ser Tyr Tyr Asn Glu Lys Phe Lys
   1 5 10 15
   Gly
<210> 25
   <211> 9
   <212> PRT
   <213> Mus musculus
<400> 25
   Gln Gln Trp Ile Ser Asn Pro Pro Thr
   1 5
<210> 26
   <211> 7
   <212> PRT
   <213> Mus musculus
<400> 26
   Gly Gly Leu Ser Phe Ala Tyr
   1 5
<210> 27
   <211> 11
   <212> PRT
   <213> Mus musculus
<400> 27
   Arg Ala Ser Glu Asn Ile Tyr Thr Tyr Leu Ala
   1 5 10
<210> 28
   <211> 5
   <212> PRT
   <213> Mus musculus
<400> 28
   Asp Tyr Trp Ile Glu
   1 5
<210> 29
   <211> 7
   <212> PRT
   <213> Mus musculus
<400> 29
   Asn Ala Lys Thr Leu Ala Glu
   1 5
<210> 30
   <211> 17
   <212> PRT
   <213> Mus musculus
<400> 30
   Glu Ile Leu Pro Gly Ser Val Ser Ile Lys Tyr Asn Glu Lys Phe Lys
   1 5 10 15
   Gly
<210> 31
   <211> 9
   <212> PRT
   <213> Mus musculus
<400> 31
   Gln His His Tyr Gly Thr Pro Tyr Thr
   1 5
<210> 32
   <211> 11
   <212> PRT
   <213> Mus musculus
<400> 32
   Ser Arg Ser Ile Tyr Asp Gly Tyr Phe Tyr Tyr
   1 5 10
<210> 33
   <211> 10
   <212> PRT
   <213> Mus musculus
<400> 33
   Ser Ala Ser Ser Ser Val Ser Tyr Met Tyr
   1 5 10
<210> 34
   <211> 5
   <212> PRT
   <213> Mus musculus
<400> 34
   Ser Tyr Gly Ile Asn
   1 5
<210> 35
   <211> 7
   <212> PRT
   <213> Mus musculus
<400> 35
   Arg Thr Ser Asn Leu Glu Ser
   1 5
<210> 36
   <211> 17
   <212> PRT
   <213> Mus musculus
<400> 36
   Glu Ile Tyr Pro Gly Ser Gly Thr Thr Phe Tyr Asn Glu Lys Phe Arg
   1 5 10 15
   Gly
<210> 37
   <211> 9
   <212> PRT
   <213> Mus musculus
<400> 37
   Gln Gln Tyr His Ser Tyr Pro Arg Thr
   1 5
<210> 38
   <211> 10
   <212> PRT
   <213> Mus musculus
<400> 38
   His Gly Gly Tyr Pro Phe Tyr Phe Asp Tyr
   1 5 10
<210> 39
   <211> 15
   <212> PRT
   <213> Mus musculus
<400> 39
   Arg Ala Ser Glu Ser Val Asp Ser Tyr Gly Asn Ser Phe Met His
   1 5 10 15
<210> 40
   <211> 7
   <212> PRT
   <213> Mus musculus
<400> 40
   Thr Ser Gly Met Gly Val Ser
   1               5
<210> 41
   <211> 7
   <212> PRT
   <213> Mus musculus
<400> 41
   Arg Ala Ser Asn Leu Glu Ser
   1               5
<210> 42
   <211> 16
   <212> PRT
   <213> Mus musculus
<400> 42
   His Ile Tyr Trp Asp Asp Asp Lys Gly Tyr Asn Pro Ser Leu Lys Ser
   1               5                   10                  15
<210> 43
   <211> 9
   <212> PRT
   <213> Mus musculus
<400> 43
   Gln Gln Ser Asn Glu Asp Pro Pro Thr
   1               5
<210> 44
   <211> 7
   <212> PRT
   <213> Mus musculus
<400> 44
   Gly Asp Val Glu Phe Asp Tyr
   1               5
<210> 45
   <211> 10
   <212> PRT
   <213> Mus musculus
<400> 45
   Ser Ala Ser Ser Ser Val Ser Tyr Met Tyr
   1 5 10
<210> 46
   <211> 5
   <212> PRT
   <213> Mus musculus
<400> 46
   Thr Tyr Gly Leu Thr
   1 5
<210> 47
   <211> 7
   <212> PRT
   <213> Mus musculus
<400> 47
   Arg Thr Ser Asn Leu Glu Ser
   1 5
<210> 48
   <211> 17
   <212> PRT
   <213> Mus musculus
<400> 48
   Glu Ile Phe Pro Gly Ser Gly Thr Thr Phe Tyr Asn Glu Lys Phe Asn
   1 5 10 15
   Asp
<210> 49
   <211> 9
   <212> PRT
   <213> Mus musculus
<400> 49
   Gln Gln Tyr His Ser Tyr Pro Arg Thr
   1 5
<210> 50
   <211> 10
   <212> PRT
   <213> Mus musculus
<400> 50
   Tyr Ser Asn Tyr Pro Tyr Tyr Phe Asp Tyr
   1 5 10
<210> 51
   <211> 112
   <212> PRT
   <213> Mus musculus
<400> 51
   Ile Val Leu Thr Gln Thr Thr Ser Ser Leu Ala Val Ser Ala Gly Glu
   1               5                   10                  15
   Lys Val Thr Met Ser Cys Lys Ser Ser Gln Ser Leu Leu Asn Ser Arg
               20                  25                  30
   Thr Pro Lys Asn Tyr Leu Ala Trp Tyr Gln Gln Lys Pro Gly Gln Ser
           35                  40                  45
   Pro Lys Leu Leu Ile Tyr Trp Ala Ser Thr Arg Lys Ser Gly Val Pro
       50                  55                  60
   Asp Arg Phe Thr Gly Ser Gly Ser Gly Thr Asp Phe Thr Leu Thr Ile
   65                  70                  75                  80
   Ser Ser Val Gln Ala Glu Asp Leu Ala Val Tyr Tyr Cys Lys Gln Ser
                   85                  90                  95
   Tyr Asn Leu Leu Thr Phe Gly Ala Gly Thr Lys Leu Glu Leu Lys Arg
               100                 105                 110
<210> 52
   <211> 119
   <212> PRT
   <213> Mus musculus
<400> 52
   Val Gln Leu Glu Glu Ser Gly Ala Glu Leu Val Lys Pro Gly Ala Ser
   1               5                   10                  15
   Val Lys Leu Ser Cys Lys Ala Ser Gly Tyr Ala Phe Thr His Tyr Tyr
               20                  25                  30
   Ile Tyr Trp Ile Lys Gln Arg Pro Gly Gln Gly Leu Glu Trp Ile Gly
           35                  40                  45
   Gly Val Asn Pro Ser Asn Gly Gly Thr His Phe Asn Glu Lys Phe Lys
       50                  55                  60
   Ser Lys Ala Thr Leu Thr Val Asp Lys Ser Ser Ser Thr Ala Tyr Met
   65                  70                  75                  80
   Gln Leu Ser Ser Leu Thr Ser Glu Asp Ser Ala Val Tyr Tyr Cys Thr
                   85                  90                  95
   Arg Ser Glu Tyr Asp Tyr Gly Leu Gly Phe Ala Tyr Trp Gly Gln Gly
               100                 105                 110
   Thr Leu Val Thr Val Ser Ala
           115
<210> 53
   <211> 111
   <212> PRT
   <213> Mus musculus
<400> 53
   Asp Ile Val Ile Thr Gln Ser Pro Ser Ser Leu Ala Val Ser Ala Gly
   1               5                   10                  15
   Glu Lys Val Thr Met Ser Cys Arg Ser Ser Gln Ser Leu Leu Asp Ser
               20                  25                  30
   Arg Thr Arg Lys Asn Tyr Leu Ala Trp Tyr Gln Gln Lys Pro Gly Gln
           35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                      80
   Ile Ser Asn Val Gln Ala Glu Asp Leu Ala Val Tyr Tyr Cys Lys Gln
                   85                  90                  95
   Ser Tyr Asn Leu Pro Thr Phe Gly Gly Gly Thr Lys Leu Glu Ile
               100                 105                 110
<210> 54
   <211> 116
   <212> PRT
   <213> Mus musculus
<400> 54
   Val Lys Leu Gln Glu Ser Gly Gly Gly Leu Val Gln Pro Gly Arg Pro
   1               5                   10                  15
   Met Lys Leu Ser Cys Val Ala Ser Gly Phe Thr Phe Ser Asp Phe Trp
               20                  25                  30
   Met Asn Trp Val Arg Gln Ser Pro Glu Lys Gly Leu Glu Trp Val Ala
           35                  40                  45
   Gln Ile Arg Asn Lys Pro Asn Asn Tyr Glu Thr Tyr Tyr Ser Asp Ser
       50                  55                  60
   Leu Lys Gly Arg Phe Thr Ile Ser Arg Asp Asp Ser Lys Ser Ser Val
   65                  70                  75                      80
   Tyr Leu Gln Met Asn Asn Leu Arg Pro Glu Asp Met Gly Ile Tyr Tyr
                   85                  90                  95
   Cys Thr Leu Gly Asn Ser Trp Phe Ala Tyr Trp Gly Gln Gly Thr Leu
               100                 105                 110
   Val Thr Val Ser
           115
<210> 55
   <211> 106
   <212> PRT
   <213> Mus musculus
<400> 55
   Gln Ile Val Leu Thr Gln Ser Pro Ala Ile Met Ser Ala Ser Pro Gly
   1               5                   10                  15
   Glu Lys Val Thr Met Thr Cys Ser Ala Ser Ser Ser Val Ser Tyr Met
               20                  25                  30
   Tyr Trp Tyr Gln Gln Lys Ser Gly Thr Ser Pro Lys Arg Trp Ile Tyr
           35                  40                  45
   Asp Thr Ser Lys Leu Ala Ser Gly Val Pro Ala Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Trp Ser Ser Asn Pro Gln Phe
                   85                  90                  95
   Gly Thr Gly Thr Lys Leu Glu Val Lys Arg
               100                 105
<210> 56
   <211> 119
   <212> PRT
   <213> Mus musculus
<400> 56
   Gln Val Lys Leu Glu Glu Ser Gly Pro Glu Leu Val Lys Pro Gly Ala
   1               5                   10                  15
   Pro Val Lys Ile Ser Cys Lys Thr Ser Gly Tyr Thr Phe Thr Asn Tyr
               20                  25                  30
   Tyr Ile Tyr Trp Val Lys Gln Arg Pro Gly Gln Gly Leu Glu Trp Ile
           35                  40                  45
   Gly Tyr Ile Tyr Pro Gly Asn Gly Gly Thr Ala Tyr Asn Gln Lys Phe
       50                  55                  60
   Lys Asp Lys Ala Thr Leu Thr Ala Asp Asn Pro Ser Asn Thr Ala Tyr
   65                  70                  75                  80
   Met Gln Leu Ser Ser Leu Thr Ser Glu Asp Ser Ala Val Tyr Leu Cys
                   85                  90                  95
   Ala Arg Arg Gly Ala Leu Gly Tyr Tyr Phe Asp Tyr Trp Gly Gln Gly
               100                 105                 110
   Thr Thr Leu Thr Val Ser Ser
           115
<210> 57
   <211> 107
   <212> PRT
   <213> Mus musculus
<400> 57
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Val Thr Tyr Met
   20 25 30
   His Trp Tyr Gln Gln Lys Ser Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Thr Met Glu Ala Glu
   65 70 75 80
   Asp Ala Ala Thr Tyr Tyr Cys Gln Gln Trp Ile Ser Asn Pro Pro Thr
   85 90 95
   Phe Gly Gly Gly Thr Lys Leu Glu Ile Lys Arg
   100 105
<210> 58
   <211> 116
   <212> PRT
   <213> Mus musculus
<400> 58
   Gln Val Gln Leu Gln Gln Ser Gly Ala Glu Leu Ala Arg Pro Gly Ala
   1 5 10 15
   Ser Val Lys Leu Ser Cys Lys Ala Ser Gly Tyr Ser Leu Thr Asn Tyr
   20 25 30
   Gly Ile Ser Trp Val Lys Gln Arg Thr Gly Gln Gly Leu Glu Trp Ile
   35 40 45
   Gly Glu Ile Tyr Arg Gly Ser Gly Asn Ser Tyr Tyr Asn Glu Lys Phe
       50                  55                  60
   Lys Gly Lys Ala Ser Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Phe Cys
                   85                  90                  95
   Ala Arg Gly Gly Leu Ser Phe Ala Tyr Trp Gly Gln Gly Thr Leu Val
               100                 105                 110
   Thr Val Ser Ala
           115
<210> 59
   <211> 107
   <212> PRT
   <213> Mus musculus
<400> 59
   Asp Ile Val Met Thr Gln Ser Thr Ala Ser Leu Ser Ala Ser Val Gly
   1               5                   10                  15
   Glu Thr Val Thr Ile Thr Cys Arg Ala Ser Glu Asn Ile Tyr Thr Tyr
               20                  25                  30
   Leu Ala Trp Tyr Gln Gln Lys Gln Gly Lys Ser Pro Gln Leu Leu Val
           35                  40                  45
   Tyr Asn Ala Lys Thr Leu Ala Glu Gly Val Pro Ser Arg Phe Ser Gly
       50                  55                  60
   Ser Gly Ser Gly Thr Gln Phe Ser Leu Arg Ile Asn Ser Leu Gln Pro
   65                  70                  75                  80
   Glu Asp Phe Gly Ser Tyr Tyr Cys Gln His His Tyr Gly Thr Pro Tyr
                   85                  90                  95
   Thr Phe Gly Gly Gly Thr Lys Leu Glu Ile Lys
               100                 105
<210> 60
   <211> 120
   <212> PRT
   <213> Mus musculus
<400> 60
   Gln Val Lys Leu Gln Glu Ser Gly Ala Glu Leu Met Lys Pro Gly Ser
   1               5                   10                  15
   Ser Val Lys Phe Ser Cys Lys Ala Ala Gly Tyr Thr Phe Thr Asp Tyr
               20                  25                  30
   Trp Ile Glu Trp Leu Lys Gln Arg Pro Gly His Gly Leu Glu Trp Ile
           35                  40                  45
   Gly Glu Ile Leu Pro Gly Ser Val Ser Ile Lys Tyr Asn Glu Lys Phe
       50                  55                  60
   Lys Gly Lys Ala Thr Phe Thr Ala Asp Thr Ser Ser Asn Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Ser Arg Ser Ile Tyr Asp Gly Tyr Phe Tyr Tyr Trp Gly Gln
               100                 105                 110
   Gly Thr Thr Leu Thr Val Ser Ser
           115                 120
<210> 61
   <211> 107
   <212> PRT
   <213> Mus musculus
<400> 61
   Asp Ile Val Met Thr Gln Ser Pro Ala Ile Met Ser Ala Ser Pro Gly
   1               5                   10                  15
   Glu Lys Val Thr Ile Ser Cys Ser Ala Ser Ser Ser Val Ser Tyr Met
               20                  25                  30
   Tyr Trp Tyr Gln Gln Lys Pro Gly Ser Ser Pro Lys Pro Trp Ile Tyr
           35                  40                  45
   Arg Thr Ser Asn Leu Ala Ser Gly Val Pro Ala Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Tyr His Ser Tyr Pro Arg Thr
                   85                  90                  95
   Phe Gly Gly Gly Thr Lys Leu Glu Ile Lys Arg
               100                 105
<210> 62
   <211> 118
   <212> PRT
   <213> Mus musculus
<400> 62
   Val Gln Leu Gln Glu Ser Gly Ala Glu Leu Ala Arg Pro Gly Ala Ser
   1               5                   10                  15
   Val Lys Leu Ser Cys Lys Ala Ser Gly Tyr Ser Phe Arg Ser Tyr Gly
               20                  25                  30
   Ile Asn Trp Val Lys Gln Arg Thr Gly Gln Gly Leu Glu Trp Ile Gly
           35                  40                  45
   Glu Ile Tyr Pro Gly Ser Gly Thr Thr Phe Tyr Asn Glu Lys Phe Arg
       50                  55                  60
   Gly Lys Ala Thr Leu Thr Ala Asp Lys Ser Ser Ser Thr Ala Tyr Met
   65                  70                  75                  80
   Gln Leu Lys Ser Leu Thr Ser Glu Asp Ser Ala Val Tyr Phe Cys Ala
                   85                  90                  95
   Arg His Gly Gly Tyr Pro Phe Tyr Phe Asp Tyr Trp Gly Gln Gly Thr
               100                 105                 110
   Thr Leu Thr Val Ser Ser
           115
<210> 63
   <211> 111
   <212> PRT
   <213> Mus musculus
<400> 63
   Asp Ile Val Leu Thr Gln Ser Thr Ala Ser Leu Ala Val Ser Leu Gly
   1               5                   10                  15
   Gln Arg Ala Thr Ile Ser Cys Arg Ala Ser Glu Ser Val Asp Ser Tyr
               20                  25                  30
   Gly Asn Ser Phe Met His Trp Tyr Gln Gln Lys Pro Gly Gln Pro Pro
           35                  40                  45
   Lys Leu Leu Ile Tyr Arg Ala Ser Asn Leu Glu Ser Gly Ile Pro Ala
       50                  55                  60
   Arg Phe Ser Gly Ser Gly Ser Arg Thr Asp Phe Thr Leu Thr Ile Asn
   65                  70                  75                  80
   Pro Val Glu Ala Asp Asp Val Ala Thr Tyr Tyr Cys Gln Gln Ser Asn
                   85                  90                  95
   Glu Asp Pro Pro Thr Phe Gly Ser Gly Thr Lys Leu Glu Ile Lys
               100                 105                 110
<210> 64
   <211> 117
   <212> PRT
   <213> Mus musculus
<400> 64
   Glu Val Gln Leu Gln Glu Ser Gly Pro Gly Ile Leu Gln Pro Ser Gln
   1               5                   10                  15
   Thr Leu Ser Leu Thr Cys Ser Phe Ser Gly Phe Ser Leu Ser Thr Ser
               20                  25                  30
   Gly Met Gly Val Ser Trp Ile Arg Gln Pro Ser Gly Lys Gly Leu Glu
           35                  40                  45
   Trp Leu Ala His Ile Tyr Trp Asp Asp Asp Lys Gly Tyr Asn Pro Ser
       50                  55                  60
   Leu Lys Ser Arg Leu Thr Ile Ser Lys Asp Thr Ser Arg Asn Gln Val
   65                  70                  75                  80
   Phe Leu Lys Ile Thr Ser Val Asp Thr Ala Asp Thr Ala Thr Tyr Tyr
                   85                  90                  95
   Cys Ala Arg Gly Asp Val Glu Phe Asp Tyr Trp Gly Gln Gly Thr Thr
               100                 105                 110
   Leu Thr Val Ser Ser
           115
<210> 65
   <211> 107
   <212> PRT
   <213> Mus musculus
<400> 65
   Gln Ile Val Leu Thr Gln Ser Pro Thr Ile Met Ser Ala Ser Pro Gly
   1               5                   10                  15
   Glu Lys Val Thr Ile Ser Cys Ser Ala Ser Ser Ser Val Ser Tyr Met
               20                  25                  30
   Tyr Trp Tyr Gln Gln Lys Pro Gly Ser Ser Pro Lys Pro Trp Ile Tyr
           35                  40                  45
   Arg Thr Ser Asn Leu Ala Ser Gly Val Pro Asp Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Tyr His Ser Tyr Pro Arg Thr
                   85                  90                  95
   Phe Gly Gly Gly Thr Lys Leu Glu Ile Lys Arg
               100                 105
<210> 66
   <211> 118
   <212> PRT
   <213> Mus musculus
<400> 66
   Val Gln Leu Gln Gln Ser Gly Ala Glu Leu Ala Arg Pro Gly Ala Ser
   1               5                   10                  15
   Val Lys Leu Ser Cys Lys Ala Ser Gly Tyr Thr Phe Thr Thr Tyr Gly
               20                  25                  30
   Leu Thr Trp Val Lys Gln Arg Thr Gly Gln Gly Leu Glu Trp Ile Gly
           35                  40                  45
   Glu Ile Phe Pro Gly Ser Gly Thr Thr Phe Tyr Asn Glu Lys Phe Asn
       50                  55                  60
   Asp Lys Ala Thr Leu Thr Ala Asp Lys Ser Ser Ser Thr Ala Tyr Met
   65                  70                  75                  80
   Gln Leu Thr Ser Leu Thr Ser Glu Asp Ser Ala Val Tyr Phe Cys Ala
                   85                  90                  95
   Arg Tyr Ser Asn Tyr Pro Tyr Tyr Phe Asp Tyr Trp Gly Gln Gly Thr
               100                 105                 110
   Thr Leu Thr Val Ser Ser
           115
<210> 67
   <211> 336
   <212> DNA
   <213> Mus musculus
<400> 67
   attgtgctga cacagactac atcctccctg gctgtgtcag caggagagaa ggtcactatg 60
   agctgcaaat ccagtcagag tctgctcaac agtagaaccc caaagaacta cttggcttgg 120
   taccagcaga aaccagggca gtctcctaaa ctgctgatct actgggcatc cactaggaaa 180
   tctggggtcc ctgatcgctt cacaggcagt ggatctggga cagatttcac tctcaccatc 240
   agcagtgtgc aggctgaaga cctggcagtt tattactgca agcaatctta taatctgctc 300
   acgttcggtg ctgggaccaa gctggagctg aaacgg 336
<210> 68
   <211> 357
   <212> DNA
   <213> Mus musculus
<400> 68
   gtccagctgg aggagtcagg ggctgaactg gtgaagcctg gggcttcagt gaaattgtcc 60
   tgcaaggctt ctggctacgc cttcacccac tactatatat actggataaa acagaggcct 120
   ggacaaggcc ttgagtggat tgggggggtt aatcctagca atggtggtac tcacttcaat 180
   gagaagttca agagcaaggc cacactgact gtagacaaat cctccagtac agcctacatg 240
   caactcagca gcctgacatc tgaggactct gcggtctatt attgtacaag atcggagtat 300
   gattacggat tggggtttgc ttactggggc caagggactc tggtcactgt ctctgca 357
<210> 69
   <211> 339
   <212> DNA
   <213> Mus musculus
<400> 69
   gacattgtga tcacacagtc tccatcctcc ctggctgtgt cagcaggaga gaaggtcact 60
   atgagctgca gatccagtca gagtctgctc gacagtagaa cccgaaagaa ctacttggct 120
   tggtaccagc agaaaccagg gcagtctcct aaactgctga tctactgggc atccactagg 180
   gaatctgggg tccctgatcg cttcacaggc agtggatctg ggacagattt cactctcacc 240
   atcagcaatg tgcaggctga agacctggca gtttattact gcaagcaatc ttataatctt 300
   ccgacgttcg gtggaggcac caagctggaa atcaaacgg 339
<210> 70
   <211> 348
   <212> DNA
   <213> Mus musculus
<400> 70
   gttaagctgc aggagtcagg aggaggcttg gtgcaacctg ggaggcccat gaaactctcc 60
   tgtgttgcct ctggattcac ttttagtgac ttctggatga actgggtccg ccagtctcca 120
   gagaaaggac tggagtgggt agcacaaatt agaaacaaac ctaataatta tgaaacgtat 180
   tattcagatt ctttgaaagg cagattcacc atctcaagag atgattccaa aagtagtgtc 240
   tacctgcaaa tgaacaactt aagacctgaa gacatgggta tctattactg tacactaggt 300
   aactcctggt ttgcttactg gggccaaggg actctggtca ctgtctct 348
<210> 71
   <211> 318
   <212> DNA
   <213> Mus musculus
<400> 71
   caaattgttc tcacccagtc tccagcaatc atgtctgcat ctccagggga gaaggtcacc 60
   atgacctgca gtgccagctc aagtgtaagt tacatgtatt ggtaccagca gaagtcaggc 120
   acctccccca aaagatggat ttatgacaca tccaaactgg cttctggagt ccctgctcgt 180
   ttcagtggca gtgggtctgg gacctcttac tctctcacaa tcagcagcat ggaggctgaa 240
   gatgctgcca cttattactg ccagcagtgg agtagtaacc cacagttcgg tactgggacc 300
   aagctggagg tgaaacgg 318
<210> 72
   <211> 357
   <212> DNA
   <213> Mus musculus
<400> 72
   caggtaaagc tggaggagtc aggacctgag ctggtgaagc ctggggctcc agtgaagata 60
   tcctgcaaga cttctggcta caccttcact aactactata tatattgggt gaaacagagg 120
   cctggacagg gcctagagtg gattggatat atttatcctg gaaatggtgg tactgcctac 180
   aatcagaaat ttaaggacaa ggccacactg actgcagaca atccctccaa cactgcctac 240
   atgcagctca gtagcctgac ctctgaggac tctgcggtct atctctgtgc aagaagaggg 300
   gctttaggat actactttga ctactggggc caaggcacca ctctcacagt ctcctca 357
<210> 73
   <211> 321
   <212> DNA
   <213> Mus musculus
<400> 73
   caaattgttc tcacccagtc tccagcaatc atgtctgcat ctccagggga gaaggtcacc 60
   atgacctgca gtgccagctc aagtgtaact tacatgcact ggtaccagca gaagtcaggc 120
   acctccccca aaagatggat ttatgacaca tccaaactgg cttctggagt ccctgctcgc 180
   ttcagtggca gtgggtctgg gacctcttac tctctcacaa tcagcaccat ggaggctgaa 240
   gatgctgcca cttattactg ccagcagtgg attagtaacc cacccacgtt cggagggggg 300
   accaagctgg aaataaaacg g 321
<210> 74
   <211> 348
   <212> DNA
   <213> Mus musculus
<400> 74
   caggttcagc tgcagcagtc tggagctgag ctggcgaggc ctggggcttc agtgaagctg 60
   tcctgcaagg cttctggata ttccctcaca aactatggta taagttgggt gaagcagaga 120
   actggacagg gccttgagtg gattggagag atttatcgtg gaagtggtaa tagttactac 180
   aatgagaagt tcaagggcaa ggcctcactg actgcagaca aatcctccag cacagcctac 240
   atgcagctca gcagcctgac atctgaggac tctgccgtct atttctgtgc aagagggggg 300
   ctatcctttg cttactgggg ccaagggact ctggtcactg tctctgca 348
<210> 75
   <211> 324
   <212> DNA
   <213> Mus musculus
<400> 75
   gatattgtga tgacccagtc tacagcctcc ctatctgcat ctgtgggaga aactgtcacc 60
   atcacatgtc gagcaagtga gaatatttac acttatttag catggtatca gcagaaacag 120
   ggaaaatctc ctcagctcct ggtctataat gcaaaaacct tagcagaagg tgtgccatca 180
   aggttcagtg gcagtggatc aggcacacag ttttctctga ggatcaacag cctgcagcct 240
   gaagattttg ggagttatta ctgtcaacat cattatggta ccccgtacac gttcggaggg 300
   gggaccaagc tggaaataaa acgg 324
<210> 76
   <211> 360
   <212> DNA
   <213> Mus musculus
<400> 76
   caggtcaagc tgcaggagtc aggagctgag ctgatgaagc ctgggtcctc agtgaagttt 60
   tcctgcaagg ctgctggcta cacattcact gactattgga tagagtggtt aaagcagagg 120
   cctggacatg gccttgagtg gattggagag attttacctg gaagtgttag tattaagtac 180
   aatgagaagt tcaagggcaa ggccacattc actgcagata catcctccaa cacagcctac 240
   atgcaactca gcagcctgac atctgaggat tctgcggtct attactgtgc aagatcaagg 300
   tctatctatg atggttactt ttattactgg ggccaaggca ccactctcac agtctcctca 360
<210> 77
   <211> 321
   <212> DNA
   <213> Mus musculus
<400> 77
   gatattgtga tgacacagtc tccagcaatc atgtctgcat ctcctgggga gaaggtcacc 60
   atttcctgca gtgccagctc aagtgtaagt tacatgtact ggtaccagca gaagccagga 120
   tcctccccca aaccctggat ttatcgcaca tccaacctgg cttctggagt ccctgctcgc 180
   ttcagtggca gtgggtctgg gacctcttac tctctcacaa tcagcagcat ggaggctgaa 240
   gatgctgcca cttattactg ccagcagtat catagttacc cacggacgtt cggtggaggc 300
   accaagctgg aaatcaaacg g 321
<210> 78
   <211> 357
   <212> DNA
   <213> Mus musculus
<400> 78
   gaggtgcagc tgcaggagtc tggagctgag ctggcgaggc ctggggcttc agtgaagctg 60
   tcctgcaagg cttctggata cagcttcaga agctatggta taaactgggt gaagcagaga 120
   actggacagg gccttgagtg gattggagag atttatcctg gaagtggtac tactttctac 180
   aatgagaagt tcaggggcaa ggccacactg actgcagaca agtcctccag cacagcctac 240
   atgcagctca aaagcctgac atctgaggac tctgccgtct atttctgtgc aagacatggt 300
   ggttacccgt tctactttga ctactggggc caaggcacca ctctcacagt ctcctca 357
<210> 79
   <211> 336
   <212> DNA
   <213> Mus musculus
<400> 79
   gatattgtgc tgacccaatc tacagcttct ttggctgtgt ctctagggca gagggccacc 60
   atatcctgca gagccagtga aagtgttgat agttatggca atagttttat gcactggtac 120
   cagcagaaac caggacagcc acccaaactc ctcatctatc gtgcatccaa cctagaatct 180
   gggatccctg ccaggttcag tggcagtggg tctaggacag acttcaccct caccattaat 240
   cctgtggagg ctgatgatgt tgcaacctac tactgtcagc aaagtaatga ggatcctccc 300
   acgttcggct cggggacaaa gttggaaata aaacgg 336
<210> 80
   <211> 351
   <212> DNA
   <213> Mus musculus
<400> 80
   gaagtgcagc tgcaggagtc aggccctggg atattgcagc cctcccagac cctcagtctg        60
   acttgttctt tctctgggtt ttcactgagc acttctggta tgggtgtgag ctggattcgt       120
   cagccttcag gaaagggtct ggagtggctg gcacacattt actgggatga tgacaagggc       180
   tataacccat ccctgaagag ccggctcaca atctccaagg atacctccag aaaccaggta       240
   ttcctcaaga tcaccagtgt ggacactgca gatactgcca catactactg tgctcgaggg       300
   gacgtcgagt ttgactactg gggccaaggc accactctca cagtctcctc a                351
<210> 81
   <211> 321
   <212> DNA
   <213> Mus musculus
<400> 81
   caaattgttc tcacccagtc tccaacaatc atgtctgcat ctccagggga gaaggtcacc        60
   atatcctgca gtgccagctc aagtgtaagt tacatgtact ggtaccagca gaagccagga       120
   tcctccccca aaccctggat ttatcgcaca tccaacctgg cttctggagt ccctgatcgc       180
   ttcagtggca gtgggtctgg gacctcttac tctctcacaa tcagcagcat ggaggctgaa       240
   gatgctgcca cttattactg ccagcagtat catagttacc cacggacgtt cggtggaggc       300
   accaagctgg aaatcaaacg g                                                 321
<210> 82
   <211> 357
   <212> DNA
   <213> Mus musculus
<400> 82
   gaagtgcagc tgcagcagtc aggagctgaa ctggcgaggc ctggggcttc agtgaagctg        60
   tcctgcaagg cttctggata caccttcaca acctatggtt taacctgggt gaagcagaga       120
   actggacagg gccttgagtg gattggagag atttttcctg gaagtggtac tactttctac       180
   aatgagaagt tcaacgacaa ggccacactg actgcagaca aatcctccag cacagcctac       240
   atgcagctca ccagcctgac atctgaggac tctgccgtct atttctgtgc aagatatagt       300
   aactacccgt actactttga ctactggggc caaggcacca ctctcacagt ctcctca          357
<210> 83
   <211> 10
   <212> PRT
   <213> Homo sapiens
<400> 83
   Lys Pro Leu Pro Glu Val Thr Asp Glu Tyr
   1 5 10
<210> 84
   <211> 18
   <212> PRT
   <213> Homo sapiens
<400> 84
   Glu Lys Glu Ala Leu Lys Lys Ile Ile Glu Asp Gln Gln Glu Ser Leu
   1 5 10 15
   Asn Lys
<210> 85
   <211> 18
   <212> PRT
   <213> Artificial
<220>
   <223> Linker peptide
<400> 85
   Glu Lys Glu Ala Leu Lys Lys Ile Ile Glu Asp Gln Gln Glu Ser Leu
   1 5 10 15
   Asn Lys